(19) Europäisches Patentamt
European Patent Office
Office européen des brevets

(11) EP 1 935 454 A1

(12) DEMANDE DE BREVET EUROPEEN

(43) Date de publication:
**25.06.2008 Bulletin 2008/26**

(21) Numéro de dépôt: **07123549.3**

(22) Date de dépôt: **19.12.2007**

(51) Int Cl.:
***A61Q 5/06*** (2006.01) ***A61Q 5/12*** (2006.01)
***A61K 8/895*** (2006.01) ***A61K 8/89*** (2006.01)
***A61K 8/11*** (2006.01)

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **20.12.2006 FR 0655681**

(71) Demandeur: **L'ORÉAL**
**75008 Paris (FR)**

(72) Inventeurs:
- **Prigent, Fanny**
**75019, Paris (FR)**
- **Simonnet, Jean-Thierry**
**94230, Cachan (FR)**

(74) Mandataire: **Le Coupanec, Pascale A.M.P.**
**Nony & Associés,**
**3 rue de Penthièvre**
**75008 Paris (FR)**

# (54) Composition comprenant des composés siliconés encapsulés

(57) La présente invention concerne une composition cosmétique destinée au soin et/ou maquillage de matière(s) kératinique(s) comprenant, dans un milieu physiologiquement acceptable, au moins un composé X, un composé Y et au moins un catalyseur, avec au moins un des composés X ou Y étant un polyorganosiloxane, et lesdits composés X et Y étant susceptibles de réagir ensemble par une réaction d'hydrosilylation en présence d'un catalyseur, avec au moins un composé parmi les composés X et Y étant présent dans ladite composition sous une forme encapsulée, ledit catalyseur étant associé audit composé X et/ou Y encapsulé.

EP 1 935 454 A1

## Description

La présente invention concerne une composition cosmétique comprenant au moins un composé X et un composé Y aptes à réagir ensemble le cas échéant en présence d'un catalyseur ou d'un peroxyde, l'un au moins des composés étant un polyorganosiloxane et l'un au moins des composés étant sous une forme encapsulée.

Les compositions considérées selon l'invention sont plus particulièrement destinées au soin et/ou au maquillage de matières kératiniques et notamment de la peau, des lèvres et des phanères.

D'une manière générale, les compositions cosmétiques sont destinées à procurer un effet esthétique et cet effet esthétique est généralement obtenu par la formation d'un film de maquillage et/ou de soin sur le support considéré comme par exemple le visage, les lèvres, les cils et les ongles.

Pour des raisons évidentes, l'optimisation des qualités de confort, de tenue dans le temps et/ou de non transfert de ces films est un souci constant dans le domaine de la cosmétique.

Il est connu que certains systèmes comprenant des composés siliconés s'avèrent capables, par simple mise en contact de ces composés le cas échéant en présence d'un catalyseur ou d'un peroxyde, de produire des films polymériques siliconés. Ainsi des composés dits composé X et composé Y, tels que définis ci-après, s'avèrent capables de polymériser in situ, à pression atmosphérique et température ambiante et de former des films avantageusement biocompatibles, non collants, légèrement opalescents voire pelables. De tels systèmes sont notamment en partie décrits dans les documents WO 01/96 450 et GB 2 407 496. Toutefois, compte-tenu de la haute réactivité de ces composés, il est impératif de les conditionner de manière séparée pour prévenir la formation prématurée d'un film.

En conséquence, la mise en oeuvre de ces systèmes dans le domaine du soin et/ou du maquillage impose un mode de conditionnement voire d'application apparenté à celui du double geste afin de garantir que le mélange des deux composés formant le système, effectué le cas échéant en présence d'un catalyseur ou d'un peroxyde, n'intervienne qu'au contact du support considéré ou ne soit réalisé que de manière extemporanée juste avant son application sur le support.

Il est clair que cette nécessité de conditionner les deux composés de manière séparée, ou le cas échéant les deux composés et le catalyseur ou le peroxyde de manière séparée, représente une contrainte tant pour le formulateur que l'utilisateur dont il serait souhaitable de s'affranchir.

La présente invention vise précisément à surmonter cette contrainte.

Les inventeurs ont ainsi constaté qu'il s'avère possible de formuler un tel système en une unique composition sans pour autant porter préjudice à la réactivité de ses deux composants.

Plus précisément, la présente invention concerne selon l'un de ses aspects, une composition cosmétique destinée au soin et/ou maquillage de matière(s) kératinique(s) comprenant dans un milieu physiologiquement acceptable au moins un composé X et un composé Y et éventuellement au moins un catalyseur ou un peroxyde, avec au moins un des composés X ou Y étant un polyorganosiloxane, et lesdits composés X et Y étant susceptibles de réagir ensemble par une réaction d'hydrosilylation en présence d'un catalyseur, ou par une réaction de condensation, ou par une réaction de réticulation en présence de peroxyde, avec au moins un composé parmi les composés X, Y et le catalyseur ou peroxyde si présent, étant présent dans ladite composition sous une forme encapsulée et dans laquelle, lorsque les composé X et Y sont susceptibles de réagir ensemble par une réaction d'hydrosilylation en présence d'un catalyseur, ledit catalyseur est associé à au moins l'un des composés X ou Y encapsulé.

Au sens de l'invention, il est entendu que les deux composés X et Y ne sont pas présents en association avec le catalyseur dans une même capsule. En effet, au regard de leur réactivité, seul leur produit de réaction serait alors présent au sein de la composition. Or, dans le cadre de l'invention et comme il ressort de la définition de la composition selon l'invention, les deux composés X et Y peuvent y être distingués l'un de l'autre.

Selon une variante préférée de l'invention, les deux composés X et Y sont tous deux présents sous des formes encapsulées séparées.

Plus particulièrement, la forme encapsulée est une nanocapsule de type coeur/écorce dont le coeur de nature lipophile est formé en tout ou partie soit d'au moins un composé X, soit d'au moins un composé Y, soit d'une huile contenant au moins un catalyseur ou peroxyde si nécessaire à l'interaction du composé X et du composé Y présents dans ladite composition, ledit catalyseur étant encapsulé avec le composé X ou le composé Y.

Selon une variante de réalisation avantageuse, l'écorce des nanocapsules comprend au moins un polymère choisi parmi les polycaprolactones.

Selon encore un autre de ses aspects, la présente invention se rapporte à l'utilisation d'une forme encapsulée d'au moins un composé X, d'une forme encapsulée d'au moins un composé Y, et/ou d'une forme encapsulée d'au moins un catalyseur ou d'un peroxyde si nécessaire à l'interaction d'un composé X avec un composé Y, pour la préparation d'une composition de soin et/ou de maquillage de matière(s) kératinique(s) comprenant un milieu physiologiquement acceptable et destinées à former après application et séchage sur ladite matière kératinique, un film polymérique issu de la réaction d'un composé X et d'un composé Y selon une réaction de type hydrosilylation en présence d'un catalyseur, ou une réaction de condensation, ou une réaction de réticulation en présence de peroxyde, avec au moins l'un des

composés X ou Y étant un polyorganosiloxane.

Selon une variante de réalisation avantageuse, lorsque le catalyseur est présent, il est contenu dans les mêmes capsules que celles contenant l'un des composés X ou le composé Y sous réserve que les composés X, Y et le catalyseur ne soient pas encapsulés ensemble dans des capsules communes.

Ainsi, selon une variante de réalisation, le catalyseur n'est pas encapsulé dans des capsules qui lui seraient spécifiques, c'est-à-dire sous une forme séparée des composés X et/ou Y.

Elle vise en outre un procédé cosmétique de revêtement pour le soin et/ou le maquillage de matière(s) kératinique(s) comprenant au moins l'application sur ladite matière kératinique d'un mélange de composés X et Y susceptibles de réagir ensemble par une réaction d'hydrosilylation en présence d'un catalyseur, ou par une réaction de condensation, ou par une réaction de réticulation en présence d'un peroxyde avec au moins un des composés X ou Y étant un polyorganosiloxane, ledit mélange étant issu d'une composition cosmétique comprenant, dans un milieu physiologiqment acceptable, au moins un tel composé X et un tel composé Y et éventuellement au moins un catalyseur ou un peroxyde, et dans laquelle au moins un composé parmi les composés X, Y et le catalyseur ou peroxyde si présent, est présent dans ladite composition sous une forme encapsulée.

Selon un mode de réalisation, la composition peut être telle que définie précédemment.

Le mélange des composés X et Y peut être obtenu soit de manière extemporanée avant application sur ladite matière kératinique, soit au moment de l'application de ladite composition sur ladite matière kératinique.

Au sens de l'invention il est entendu que le mélange ainsi formé comprend des composés X et/ou Y sous une forme n'ayant pas encore réagi et non pas exclusivement sous la forme de leur produit de réaction par hydrosilylation, par polycondensation et/ou par réticulation en présence d'un peroxyde.

Ainsi, la formation du produit de réaction selon l'invention, peut être soit réalisée directement sur la surface de la matière kératinique devant être traitée, soit initiée juste avant application par mélange extemporané des composés X et Y dans des conditions propices à leur interaction, la formation du produit de réaction étant dans ce dernier cas finalisée en surface de la matière kératinique.

Pour des raisons évidentes, et au regard de la grande réactivité des composés X et/ou Y, il est en effet nécessaire que leur mise en oeuvre soit réalisée dans des conditions propices à la maniabilité de la composition le (ou les) contenant notamment en vue de son étalement par exemple. Le procédé conforme à l'invention met donc en oeuvre une composition contenant des composés X et Y, et donc non figée sous la forme du film final attendu et résultant de la réaction de la totalité de X et/ou de la totalité de Y.

Comme il ressort des exemples figurant ci-après, les inventeurs ont constaté que les compositions telles que décrites précédemment s'avèrent stables dans le temps et demeurent efficaces pour former un film polymérique siliconé. Lorsque ces compositions sont étalées sous la forme d'un film sur un support, par exemple une matière kératinique, la ou les formes encapsulées se cassent au séchage et les composés X et Y mis alors en contact le cas échéant en présence d'un catalyseur, réagissent entre eux pour former un film pelable, non collant et légèrement opalescent. Avantageusement, les compositions selon l'invention permettent de différer la réaction des composés X et Y qui s'effectue uniquement au moment du séchage de la composition appliquée sous forme d'un film sur le support considéré.

## FORME ENCAPSULEE

Dans les domaines de la cosmétique et de la pharmacie, il est fréquent de formuler des actifs sous des formes encapsulées dans un souci notamment de leur garantir une stabilité accrue au sein d'une composition.

Dans le cadre de la présente invention, sont plus particulièrement considérées les formes particulaires de type coeur/écorce dites encore microcapsules ou nanocapsules dont l'écorce est de nature polymérique et le coeur est de nature lipophile et plus particulièrement contient au moins une huile. Le composé X ou Y associé le cas échéant à un catalyseur, est encapsulé au sein de ce coeur lipophile.

De nombreuses techniques sont à ce jour disponibles pour préparer ce type de microcapsules ou nanocapsules.

La voie d'accès la plus classique repose sur la technique d'émulsification et implique la synthèse simultanée de l'écorce polymérique par réticulation des monomères correspondants en présence de l'actif lipophile à encapsuler. Par exemple, le document FR 1 583 243 décrit un procédé de fabrication de microcapsules contenant des gouttelettes d'huile dont la taille varie de 0,1 à plusieurs microns et qui utilise des agents de réticulation chimiques. Cette technique est également illustrée dans le document WO 02/09862. La demande WO 93/08908 utilise cette même technique pour préparer des nanocapsules dont l'écorce dérive de la réticulation de protéines. La demande WO 02/051536 décrit quant à elle des suspensions de particules de taille comprise entre 70 nm et 5 $\mu$m, obtenues par réticulation de monomères, cette réticulation étant initiée par exposition de la suspension à une irradiation UV.

Ces techniques peuvent être appliquées pour préparer des formes encapsulées de composés X et Y encapsulées le cas échéant avec un catalyseur.

Toutefois, selon un mode préféré, les formes encapsulées considérées selon l'invention, sont des nanocapsules et sont notamment obtenues par une technique dite basculement de solvant et qui consiste à solubiliser le composé X

ou Y, et le cas échéant le catalyseur ou le peroxyde, et le polymère devant constituer l'enveloppe de la nanocapsule, au sein d'un solvant. La formation des nanocapsules est initiée par addition, sous agitation, à cette solution d'un solvant annexe incapable de solubiliser le polymère, le composé X ou Y le catalyseur et le peroxyde mais en revanche miscible avec le premier solvant. Cette technique est notamment illustrée dans les documents EP 274 961 et EP 1 552 820.

Selon une variante préférée, la technique retenue est plus particulièrement celle décrite dans le document EP 1 552 820 et qui implique la formation d'une pré-émulsion.

Plus précisément, ce procédé comprend :

a) la formation d'une phase organique liquide monophasique comprenant au moins :

- un solvant organique, présentant une température d'ébullition inférieure ou égale à 100 °C,
- un polymère soluble dans ledit milieu solvant,
- une huile contenant au moins un composé X ou Y ou un catalyseur ou peroxyde si nécessaire à l'interaction du composé X avec le composé Y conjointement présents, et
- un tensioactif non ionique,

b) la préparation d'une phase aqueuse contenant au moins un tensioactif non ionique et le cas échéant un tensioactif ionique,

c) la dispersion de la phase organique (a) dans la phase aqueuse (b) de manière à obtenir une pré-émulsion,

d) la soumission de la pré-émulsion obtenue en (c) à une homogénéisation de manière à obtenir la formation d'une dispersion de nanocapsules de taille moyenne inférieure ou égale à 1 μm et contenant dans leur coeur lipophile au moins un composé X ou Y ou le catalyseur ou peroxyde nécessaire à l'interaction du composé X avec le composé Y.

Un procédé analogue peut également permettre la formation d'une dispersion de nanocapsules contenant dans leur coeur lipophile au moins un composé X ou Y encapsulé avec au moins un catalyseur nécessaire à l'interaction du composé X avec le composé Y.

Les particules peuvent être récupérées sous la forme d'une dispersion aqueuse après évaporation de la phase organique et le cas échéant d'une partie de l'eau et mises en oeuvre sous cette forme pour la préparation de compositions cosmétiques conformes à l'invention.

L'écorce des nanocapsules mises en oeuvre selon l'invention est de nature polymérique.

Plus particulièrement, l'écorce des nanocapsules mises en oeuvre selon l'invention, est de nature polymérique, non réticulée, non hydrosoluble et non soluble dans le coeur des nanocapsules.

D'une manière générale, tous les polymères, d'origine naturelle ou synthétique, solubles dans un solvant non miscible à l'eau et notamment ceux ayant un point de fusion inférieur au point d'ébullition de l'eau à la pression atmosphérique (100 °C), peuvent convenir.

Ces polymères peuvent être biodégradables, comme par exemple les polyesters, ou non.

A titre illustratif des polymères convenant à l'invention, on peut notamment citer :

- les polymères de cyanoacrylate d'alkyle en $C_2$-$C_{12}$ et, en particulier, en $C_2$-$C_6$ avec le radical alkyle pouvant en particulier choisi parmi les radicaux éthyle, n-butyle, hexyle, isobutyle et isohexyle,
- les polymères formés par les poly-L-lactides, les poly-DL-lactides, les polyglycolides et les copolymères correspondants, tels que les copoly(DL-lactides et glycolides), copoly(glycolides et caprolactones) et similaires,
- les polycaprolactones, telles que les polycaprolactones ayant un point de fusion variant de 40 à 70 °C et de poids moléculaire compris entre 2 000 et 100 000, comme par exemple celles commercialisées par la société Solvay sous les références commerciales CAPA 6806 (PM de 80000), CAPA 6100 (PM de 10000), CAPA 6506 (PM 50000), CAPA 6250 (PM de 25000), CAPA 2803 (PM de 8000), CAPA 2403 D (PM de 4000),
- les polymères de l'acide 3-hydroxy butyrique,
- les copolymères de chlorure de vinyle et d'acétate de vinyle, par exemple ceux commercialisés sous la dénomination RHODOPAS AX 8515® par la société RHONE POULENC,
- les copolymères d'acide et d'ester méthacrylique, notamment d'acide méthacrylique et d'ester d'acide méthacrylique, par exemple ceux commercialisés sous la dénomination EUDRAGIT L 100® par la société ROHM PHARMA,
- l'acéto-phtalate de polyvinyle,
- l'acéto-phtalate de cellulose,
- le copolymère polyvinylpyrrolidone-acétate de vinyle,
- les polyéthylènevinylacétates,
- les polyacrylonitriles,
- les polyacrylamides,
- les polyéthylèneglycols,

- les poly-(méthacrylate d'hydroxyalkyle en $C_1$ à $C_4$) et en particulier les poly-(méthacrylate d'hydroxyéthyle),
- les dérivés de cellulose tels que les esters de cellulose et d'au moins un acide carboxylique en $C_1$ à $C_4$, notamment des esters de cellulose mixtes de deux types d'acides carboxyliques,
- le polystyrène et les copolymères de styrène et d'anhydride maléique, les copolymères de styrène et d'acide acrylique, les terpolymères séquencés styrène éthylène/butylène-styrène, les terpolymères séquencés styrène-éthylène/propylène-styrène,
- les oligomères styrène alkylalcool,
- les terpolymères d'éthylène, d'acétate de vinyle et d'anhydride maléique,
- les polyamides,
- les polyéthylènes,
- les polypropylènes,
- les organopolysiloxanes dont les polydiméthylsiloxanes,
- les poly (alkylène adipate) qui englobent à la fois les homopolymères d'acide adipique et d'un alcane-diol et les copolymères de type poly(ester éther), linéaires ou ramifiés, obtenus à partir d'acide adipique et d'un ou de plusieurs alcane-diols et/ou éthers-diols et/ou triols ; les alcane-diols utilisés pour la préparation desdits poly(alkylène adipate) peuvent être des alcane-diols en $C_2$-$C_6$ à chaîne linéaire ou ramifiée choisis parmi l'éthylèneglycol, le propylèneglycol, le 1,3-propanediol, le 1,4-butanediol, le 1,5-pentanediol, le 1,6-hexanediol et le néopentylglycol. Les éther-diols sont des di-, tri-ou tétra-(alkylène en $C_2$-$C_4$)-glycols tels que le diéthylèneglycol, le triéthylèneglycol, le tétraéthylèneglycol, le dipropylèneglycol, le tripropylèneglycol, le tétrapropylèneglycol, le dibutylèneglycol, le tributylèneglycol ou le tétrabutylèneglycol ; à titre d'exemple, on peut plus particulièrement citer les Fomrez® commercialisés par la société Witco et les Poly(éthylène) adipate de la société Scientific Polymer Products ; on peut également citer à titre d'exemple, l'Eastar Bio® de Eastman Chemical (poly (tetraméthylène Adipate co téréphtalate) et l'Ecoflex F BX 7011® de BASF ( terpolymère Butanediol 1,4 acide terephtalique et acide adipique),
- les polyesters polyol obtenus par polycondensation d'un acide dicarboxylique aliphatique avec au moins deux alcane-diols ou avec au moins un alcane-diol et au moins un hydroxyalkyl alcane-diol avec l'acide dicarboxylique aliphatique pouvant être l'acide adipique (acide hexane-1,6-dioïque) ; de tels polymères sont notamment décrits dans le document FR 2 836 381,
- les polymères siliconés de polysilsesquioxane, notamment un polyalkylsilsesquioxane de formule : $(R\text{-}SiO_{3/2})_{xo}$ dans laquelle R représente un radical hydrocarboné, saturé ou insaturé, linéaire, ramifié ou cyclique; par exemple du type $-C_nH_{2n+1}$, avec n étant un entier allant de 1 à 20, notamment un radical méthyle, éthyle, propyle, butyle, pentyle, hexyle, heptyle, octyle, nonyle, décyle, dodécyle, tridécyle, tétradécyle, hexadécyle, octadécyle et eicosyle; ou encore un groupe aryle, notamment phényle ou tolyle; un groupe cycloalkyle, notamment cyclobutyle, cyclopentyle ou cyclohexyle; un groupe alcényle, notamment vinyle ou allyle; un groupe aralkyle, notamment 2-phényléthyle ou benzyle; R pouvant également comprendre un ou plusieurs atomes d'halogène, notamment de fluor ou de chlore; de préférence R est un radical méthyle, éthyle, propyle ou phényle;et x est le nombre de motifs, et peut être compris entre 1 et 10, notamment 1 à 4 ; à titre d'exemple, on peut citer la Belsil PMS MK® de Wacker ou la Resin KR 220® de Shin Etsu,
- les polyesters dendritiques à fonction hydroxyle terminale notamment ceux décrits dans le document FR 2 790 405,
- les polymères hydrodipersibles mais néanmoins solubles dans des solvants non miscibles à l'eau comme par exemple : les polyesters, poly(ester amides), polyuréthannes et copolymères vinyliques portant des fonctions acide carboxylique et/ou sulfonique et en particulier ceux décrits dans le document FR 2 787 729,
- les copolymères blocs insolubles dans l'eau à température ambiante et solides à température ambiante, ayant au moins un bloc d'un des polymères précédents, et
- leurs mélanges.

Selon un mode de réalisation, l'écorce des nanocapsules peut comprendre au moins un polymère choisi parmi :

- les polymères de cyanoacrylate d'alkyle en $C_2$-$C_{12}$,
- les poly-L-lactides, les poly-DL-lactides, les polyglycolides et les copolymères correspondants,
- les polycaprolactones,
- les polymères de l'acide 3-hydroxy butyrique,
- les copolymères de chlorure de vinyle et d'acétate de vinyle,
- l'acéto-phtalate de polyvinyle,
- l'acéto-phtalate de cellulose,
- le copolymère polyvinylpyrrolidone-acétate de vinyle,
- les polyéthylènevinylacétates,
- les copolymères d'acide et d'ester méthacrylique,
- les polyacrylonitriles,

- les polyacrylamides,
- les polyéthylèneglycols,
- les poly-(méthacrylate d'hydroxyalkyle en $C_1$ à $C_4$),
- les dérivés de cellulose,
- le polystyrène et ses copolymères,
- les oligomères styrène alkylalcool,
- les terpolymères d'éthylène, d'acétate de vinyle et d'anhydride maléique,
- les polyamides,
- les polyéthylènes,
- les polypropylènes,
- les organopolysiloxanes,
- les poly (alkylène adipate),
- les polyesters polyol,
- les polymères siliconés de polysilsesquioxane,
- les polyesters dendritiques à fonction hydroxyle terminale, et leurs mélanges.

Ces polymères ou copolymères peuvent posséder un poids moléculaire en poids compris entre 1000 et 500 000 et en particulier entre 1500 et 100 000.

Le polymère de l'écorce des nanocapsules peut notamment être choisi parmi :

- les polycaprolactones,
- l'acéto-phtalate de polyvinyle,
- l'acéto-phtalate de cellulose,
- les copolymères d'acide et d'ester méthacrylique,
- les dérivés de cellulose,
- le polystyrène et ses copolymères,
- les polyamides,
- les organopolysiloxanes,
- les poly (alkylène adipate), et
- leurs mélanges.

Conviennent tout particulièrement à l'invention, les poly(alkylène adipate), les organo polysiloxanes, les polycaprolactones, l'acétophtalate de cellulose, l'acétobutylate de cellulose, les esters de cellulose, le polystyrène, et ses dérivés.

Les polycaprolactones sont préférés.

Bien entendu, l'homme du métier est à même, de par ses connaissances, d'ajuster le poids moléculaire du polymère sélectionné vis-à-vis de sa concentration dans le solvant afin d'avoir une viscosité du mélange compatible avec une émulsification satisfaisante.

En ce qui concerne le coeur lipophile, il contient le composé X, le composé Y, ou le catalyseur ou peroxyde formulé dans au moins une huile si nécessaire à l'interaction du composé X et du composé Y, ou encore le catalyseur encapsulé avec le composé X et/ou Y, sous réserve que les composés X, Y et le catalyseur ne soient pas réunis dans une même capsule.

Au sens de la présente invention, le terme « huile » désigne toutes les substances huileuses, liquides à partir de 40 °C, naturelles, végétales ou animales, ou synthétiques ayant ou non une ou plusieurs activités biologiques avérées et insolubles dans l'eau (moins de 2 % en poids à température ambiante).

Selon un mode de réalisation, les nanocapsules conformes à l'invention ont un diamètre moyen ("ou taille") inférieur à 1 $\mu$m, de préférence inférieur ou égal à 500 nm, par exemple compris entre 200 et 500 nm, de préférence inférieur ou égal à 350 nm, par exemple compris entre 200 et 350 nm.

Ce diamètre peut notamment être contrôlé selon une technique de mesure reposant sur la diffusion dynamique de la lumière avec par exemple un appareil BI90+® de chez Broohaven Instrument ou Malvern Mastersizer 2000 (adapté par les ∅ sup. à 2 $\mu$m).

Selon une variante de l'invention les formes encapsulées des composés X ou composés Y et/ou d'un catalyseur peuvent être enrobées d'une phase lamellaire.

On entend par « phase lamellaire » (phase D selon EKWALL), une phase cristal liquide à symétrie plane, comprenant plusieurs bicouches d'amphiphile disposées en parallèle et séparées par un milieu liquide qui est généralement de l'eau.

Une définition plus précise de cette dénomination est donnée dans Ekwall (1968), Adv. Liq. Cryst. (Brown G.H., Ed.), Chap. 1, 14. Cette phase possède une texture caractéristique au microscope en lumière polarisée dont on pourra

trouver une description plus précise dans Roservear (1968), JSCC, 19, 581. et dans Lachampt et Vila (1969), Revue Française des Corps Gras, n° 2, 87-111.

Il est souvent souhaitable ou nécessaire de pourvoir les nanocapsules d'un enrobage dit « lamellaire ». Il s'agit d'une structure organisée en un ou plusieurs feuillet(s) lipidique(s) constitué(s) chacun d'une bicouche de molécules amphiphiles semblable à celle des membranes biologiques. L'enveloppe polymérique des nanocapsules selon l'invention peut être ainsi entourée d'un enrobage lamellaire ayant une structure organisée en un ou plusieurs feuillet(s) constitué (s) chacun d'une double couche de molécules amphiphiles constituant un agent d'enrobage. Cet enrobage, outre sa fonction d'ajustement de la taille des nanocapsules, améliore l'étanchéité des nanocapsules vis-à-vis d'une fuite de l'actif vers une autre phase lipidique de la composition.

Les agents d'enrobage sont des agents tensioactifs à caractère hydrophobe, solubles dans la phase organique utilisée dans le procédé décrit ci-dessus et qui sont capables, en présence d'eau, de former les doubles couches lipidiques décrites ci-dessus. Dans le procédé d'encapsulation de principes actifs, utilisé par la demanderesse, cet agent d'enrobage est dissous dans la phase organique contenant le polymère et la phase lipidique.

On peut citer à titre d'exemple de tels agents d'enrobage, les phospholipides tels que la lécithine comme décrit dans le document EP-A-447 318 ; certains polycondensats d'oxyde d'éthylène et d'oxyde de propylène, comme les produits vendus sous la dénomination PLURONIC® par la société BASF tels que PLURONIC® L121 ou sous la dénomination SYNPERONIC® par la société ICI; ou les agents tensioactifs siliconés (silicones comportant au moins une chaîne oxyéthylénée et/ou oxypropylénée) capables de former des structures lamellaires, tels que ceux décrits dans les documents US-A-5,364,633 et US-A-5,411,744 et utilisés dans la demande de brevet FR-A-2,742,677, par exemple ceux vendus par la société DOW CORNING sous les dénominations DC 5329, DC 7439-146, DC 2-5695 et Q4-3667 ; et leurs mélanges.

Les phases lamellaires peuvent également comprendre un lipide amphiphile ionique. Ce dernier peut être choisi parmi les lipides anioniques et les lipides cationiques.

Les lipides amphiphiles convenant à la mise en oeuvre de l'invention peuvent être :

- des lipides anioniques neutralisés, comme par exemple le sel disodique de l'acide N-stéaroyl L-glutamique commercialisé sous la dénomination Acylglutamate HS21 par la société AJINOMOTO,
- des lipides amphotères, de préférence, des phospholipides, et
- les lipides amphiphiles cationiques pouvant être utilisés comme lipides amphiphiles ioniques peuvent être plus particulièrement choisis dans le groupe formé par les sels d'ammonium quaternaire, les amines grasses et leurs sels.

En ce qui concerne le protocole opératoire pour préparer des nanocapsules convenant à l'invention, l'homme de l'art pourra se reporter notamment à l'enseignement du document EP 1 552 820 cité précédemment. Le choix des tensioactifs requis ainsi que la mise en oeuvre du procédé fait appel aux connaissances de l'homme de l'art.

## COMPOSES X ET Y

Par composé siliconé, on entend un composé polyorganosiloxane, c'est-à-dire comprenant au moins deux unités organosiloxanes, par exemple au moins 5 unités organosiloxane, notamment au moins 10 unités organosiloxane. Selon un mode de réalisation particulier, l'un au moins des composés X et Y, ou les composés X et les composés Y sont siliconés. Les composés X et Y peuvent être aminés ou non aminés.

Selon un autre mode de réalisation, au moins un des composés X et Y est un polymère dont la chaîne principale est formée majoritairement d'unités organosiloxanes. Parmi les composés siliconés cités ci-après, certains peuvent présenter à la fois des propriétés filmogènes et adhésives, selon par exemple leur proportion en silicone ou selon qu'on les utilise en mélange avec un additif particulier. Il est par conséquent possible de moduler les propriétés filmogènes ou les propriétés adhésives de tels composés selon l'utilisation envisagée, c'est en particulier le cas pour les silicones élastomères réactives dites " room temperature vulcanization".

Les composés X et Y peuvent réagir ensemble à une température variant entre la température ambiante et 180°C. Avantageusement les composés X et Y sont susceptibles de réagir ensemble à température ambiante (20 ± 5°C) et pression atmosphérique, ou avantageusement en présence d'un catalyseur, par une réaction d'hydrosilylation ou une réaction de condensation, ou une réaction de réticulation en présence d'un peroxyde.

### Groupes polaires

Selon un mode de réalisation particulier, l'un au moins des composés X et Y, par exemple le composé X, est porteur d'au moins un groupe polaire susceptible de former au moins une liaison hydrogène avec les matières kératiniques.

Par groupe polaire, on entend un groupe comportant des atomes de carbone et d'hydrogène dans sa structure

chimique et au moins un hétéroatome (tel que O, N, S et P), tel que ledit groupe est apte à établir au moins une liaison hydrogène avec les matières kératiniques .

Des composé porteurs d'au moins un groupement apte à établir une liaison hydrogène sont particulièrement avantageux, car ils apportent aux compositions les contenant une meilleure adhérence sur les matières kératiniques, grâce à l'aptitude de ces groupements à établir une liaison hydrogène avec les matières kératiniques.

Le ou les groupes polaires porté(s) par au moins l'un des composés X et Y est/sont apte(s) à établir une liaison hydrogène, et comporte(nt) soit un atome d'hydrogène lié à un atome électronégatif, soit un atome électronégatif comme par exemple l'atome d'oxygène, d'azote ou de soufre. Lorsque le groupe comporte un atome d'hydrogène lié à un atome électronégatif, l'atome d'hydrogène peut interagir avec un autre atome électronégatif porté, par exemple par une autre molécule, telle que la kératine, pour former une liaison hydrogène. Lorsque le groupement comporte un atome électronégatif, l'atome électronégatif peut interagir avec un atome d'hydrogène lié à un atome électronégatif porté, par exemple par une autre molécule, telle que la kératine, pour former une liaison hydrogène.

Avantageusement, ces groupes polaires peuvent être choisis parmi les groupes suivants :

- acide carboxylique -COOH,
- alcools, tels que : $-CH_2OH$ ou -CH(R)OH, R étant un radial alkyle comprenant de 1 à 6 atomes de carbone,
- amino de formule $-NR_1R_2$, dans laquelle les $R_1$ et $R_2$ identiques ou différents représentent un radial alkyle comprenant de 1 à 6 atomes de carbone ou l'un des R1 ou R2 désigne un atome d'hydrogène,
- pyridino,
- amido de formule -NH-COR' ou -CO-NH-R' dans laquelle R' représente un atome d'hydrogène ou un radial alkyle comprenant de 1 à 6 atomes de carbone
- pyrrolidino choisi de préférence parmi les groupes de formule :

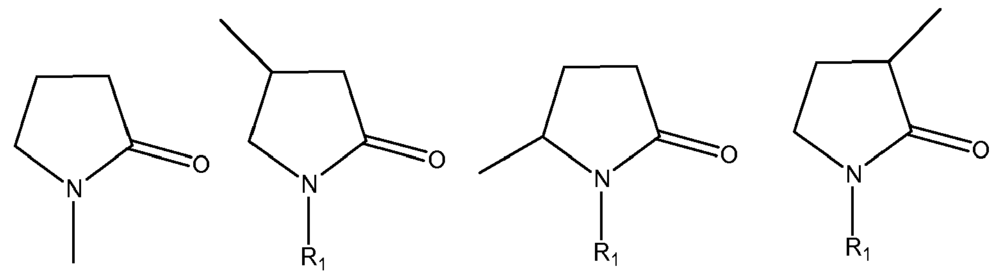

$R_1$ étant un radial alkyle comprenant de 1 à 6 atomes de carbone,

- carbamoyle de formule -O-CO-NH-R' ou -NH-CO-OR', R' étant tel que défini ci-dessus,
- thiocarbamoyle, tel que -O-CS-NH- R' ou -NH-CS-O R', R' étant tel que défini ci-dessus,
- uréyle tel que -NR' $-CO-N(R')_2$, les R' identiques ou différents étant tels que définis ci-dessus,
- sulfonamido tel que -N R'$-S(=O)_2-$ R', R' répondant à la définition ci-dessus.

De préférence, ces groupes polaires sont présents à une teneur inférieure ou égale à 10 % en poids par rapport au poids de chaque composé X ou Y, de préférence inférieure ou égale à 5 % en poids, par exemple en une teneur allant de 1 à 3 % en poids

Le ou les groupes polaires peuvent être situés dans la chaîne principale du composé X et/ou Y ou peuvent être pendants à la chaîne principale ou situés aux extrémités de la chaîne principale du composé X et/ou Y.

**1- Composés X et Y susceptibles de réagir par hydrosilylation**

Selon un mode de réalisation, l'invention concerne une composition cosmétique destinée au soin et/ou au maquillage de matière(s) kératinique(s) comprenant dans un milieu physiologiquement acceptable au moins un composé X, un composé Y, et au moins un catalyseur, avec au moins un des composés X ou Y étant un polyorganosiloxane, et lesdits composés X et Y étant susceptibles de réagir ensemble par une réaction d'hydrosilylation en présence d'un catalyseur, avec au moins un composé parmi les composés X et Y étant présent dans ladite composition sous une forme encapsulée, ledit catalyseur étant associé à au moins l'un des composés X ou Y encapsulé.

Selon ce mode de réalisation, les composés X et Y sont susceptibles de réagir par hydrosilylation en présence d'un catalyseur, cette réaction pouvant être de manière simplifiée schématisée comme suit :

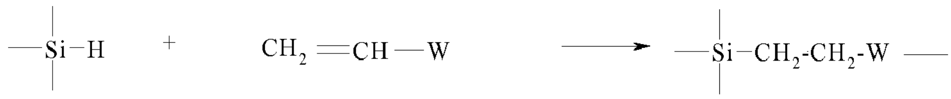

avec W représentant une chaîne carbonée et/ou siliconée contenant un ou plusieurs groupements aliphatiques insaturés.

Dans ce cas, le composé X peut être choisi parmi les composés siliconés comprenant au moins deux groupements aliphatiques insaturés. A titre d'exemple, le composé X peut être un polyorganosiloxane comprenant une chaîne principale siliconée dont les groupements aliphatiques insaturés sont pendants à la chaîne principale (groupe latéral) ou situés aux extrémités de la chaîne principale du composé (groupe terminal). On appellera, dans la suite de la description, ces composés particuliers des polyorganosiloxanes à groupements aliphatiques insaturés.

Selon un mode de réalisation, le composé X et/ou le composé Y est porteur d'au moins un groupe polaire, tel que décrit ci-dessus, susceptible de former au moins une liaison hydrogène avec les matières kératiniques. Ce groupe polaire est avantageusement porté par le composé X qui comprend au moins deux groupements aliphatiques insaturés.

Selon un mode de réalisation, le composé X est choisi parmi les polyorganosiloxanes comprenant au moins deux groupements aliphatique insaturés, par exemple deux ou trois groupements vinyliques ou allyliques, liés chacun à un atome de silicium.

Selon un mode de réalisation avantageux, le composé X est choisi parmi les polyorganosiloxanes comprenant des unités siloxanes de formule :

$$R_mR'SiO_{\frac{(3-m)}{2}} \quad (I)$$

dans laquelle :

- R représente un groupe hydrocarboné monovalent, linéaire ou cyclique, comprenant de 1 à 30 atomes de carbone, de préférence de 1 à 20, et mieux de 1 à 10 atomes de carbone, comme par exemple un radical alkyle à chaîne courte, comprenant par exemple de 1 à 10 atomes de carbone, en particulier un radical méthyle ou encore un groupement phényle, de préférence un radical méthyle,
- m est égal à 1 ou 2 et
- R' représente :

  o un groupement hydrocarboné aliphatique insaturé comprenant de 2 à 10, de préférence de 3 à 5 atomes de carbone comme par exemple un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène ; on peut citer comme groupement R' les groupements vinyle, allyle et leurs mélanges ; ou
  o un groupement hydrocarboné cyclique insaturé comprenant de 5 à 8 atomes de carbone comme par exemple un groupe cyclohexènyle.

De préférence R' est un groupement hydrocarboné aliphatique insaturé, de préférence un groupe vinyle.

Selon un mode de réalisation, R représente un radical alkyle comprenant de 1 à 10 atomes de carbone ou encore un groupement phényle, et de préférence un radical méthyle, et R' est un groupe vinyle.

Selon un mode de réalisation particulier, le polyorganosiloxane comprend également des unités de formule :

$$R_nSiO_{\frac{(4-n)}{2}} \quad (II)$$

dans laquelle R est un groupe tel que défini plus haut, et n est égal à 1, 2 ou 3.

Selon une variante, le composé X peut être une résine de silicone comprenant au moins deux insaturations éthyléniques, ladite résine étant apte à réagir avec le composé Y par hydrosilylation en présence d'un catalyseur. On

peut citer par exemple les résines de type MQ ou MT portant elle-même des extrémités réactives insaturées $-CH=CH_2$.

Ces résines sont des polymères d'organosiloxanes réticulés.

La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

La lettre M représente l'unité monofonctionelle de formule $(CH_3)_3SiO_{1/2}$, l'atome de silicium étant relié à un seul atome d'oxygène dans le polymère comprenant cette unité.

La lettre D signifie une unité difonctionnelle $(CH_3)_2SiO_{2/2}$ dans laquelle l'atome de silicium est relié à deux atomes d'oxygène

La lettre T représente une unité trifonctionnelle de formule $(CH_3)SiO_{3/2}$.

Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl ou bien encore un groupe hydroxyle.

Enfin, la lettre Q signifie une unité tetrafonctionnelle $SiO_{4/2}$ dans laquelle l'atome de silicium est lié à quatre atomes d'hydrogène eux mêmes liés au reste du polymère. Comme exemples de telles résines, on peut citer les résines de silicone MT telles que les poly(phényl-vinylsilsesquioxane) comme celle commercialisées sous la référence SST-3PV1 par la société Gelest.

De préference, les composés X comprennent de 0,01 à 1 % en poids de groupes aliphatiques insaturés.

Avantageusement, le composé X est choisi parmi les polyorganopolysiloxanes, notamment ceux comprenant les unités siloxanes (I) et éventuellement (II) décrites précédemment.

Le composé Y comprend de préférence au moins deux groupes Si-H (groupes hydrogénosilanes) libres.

Le composé Y peut être avantageusement choisi parmi les polyorganosiloxanes comprenant au moins une unité alkylhydrogènosiloxane de formule suivante :

$$R_pHSiO_{\frac{(3-p)}{2}} \quad \text{(III)}$$

dans laquelle :

R représente un groupe hydrocarboné monovalent, linéaire ou cyclique, comprenant de 1 à 30 atomes de carbone, comme par exemple un radical alkyle ayant de 1 à 30 atomes de carbone, de préférence de 1 à 20 et mieux de 1 à 10 atomes de carbone, en particulier un radical méthyle, ou encore un groupement phényle et p est égal à 1 ou 2. De préférence R est un groupement hydrocarboné, de préférence le méthyle.

Ces composés Y polyorganosiloxanes à unités alkylhydrogènosiloxanes peuvent comprendre en outre des unités de formule :

$$R_nSiO_{\frac{(4-n)}{2}} \quad \text{(II)}$$

telles que définies plus haut.

Le composé Y peut être une résine de silicone comprenant au moins un motif choisi parmi les motifs M, D, T, Q tels que définis ci-dessus et comprenant au moins un groupe Si-H tel que les poly(méthyl-hydridosilsesquioxane) commercialisées sous la référence SST-3MH1.1 par la société Gelest.

De préférence, ces composés Y polyorganosiloxanes comprennent de 0,5 à 2,5% en poids de groupes Si-H.

Avantageusement, les radicaux R représentent un groupement méthyle dans les formules (I), (II), (III) ci-dessus.

De préférence, ces polyorganosiloxanes Y comprennent des groupes terminaux de formule $(CH_3)_3SiO_{1/2}$.

Avantageusement, les polyorganosiloxanes Y comprennent au moins deux unités alkylhydrogènosiloxane de formule $-(H_3C)(H)SiO-$ et comprennent éventuellement des unités $-(H_3C)_2SiO-$.

De tels composés polyorganosiloxanes Y à groupements hydrogénosilane sont décrits par exemple dans le document EP 0465744.

Selon une variante, le composé X est choisi parmi les oligomères ou polymères organiques (par organique,

on entend des composés dont la chaîne principale est non siliconée, de préférence des composés ne comprenant pas d'atomes de silicium) ou parmi les polymères ou oligomères hybrides organique/silicone, lesdits oligomères ou polymères portant au moins 2 groupements aliphatiques insaturés réactifs, le composé Y étant choisi parmi les polyorganosiloxanes Y à groupements hydrogénosilane cités ci-dessus.

Selon un mode de réalisation, les composés X organiques ou hybrides organique/silicone portant au moins 2 groupements aliphatiques insaturés réactifs, portent au moins un groupe polaire tel que décrit plus haut.

Le composé X, de nature organique, peut alors être choisi parmi les polymères ou oligomères vinyliques, (méth)acryliques, les polyesters, les polyuréthanes et/ou les polyurées, les polyéthers, les perfluoropolyéthers, les polyoléfines telles que le polybutène, le polyisobutylène, les dendrimères ou les polymères hyper-ramifiés organiques, ou leurs mélanges.

En particulier, le polymère organique ou la partie organique du polymère hybride peut être choisi parmi les polymères suivants :

a) les polyesters à insaturation(s) éthylénique(s) :

Il s'agit d'un groupe de polymères de type polyester présentant au moins 2 doubles liaisons éthyléniques, réparties de manière aléatoire dans la chaîne principale du polymère. Ces polyesters insaturés sont obtenus par polycondensation d'un mélange :

- de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturations éthyléniques tels que les dimères des acides oléique ou linoléique décrits dans la demande EP-A-959 066 (paragraphe [0021]) commercialisés sous les dénominations Pripol® par la société Unichema ou Empol® par la société Henkel, tous ces diacides devant être exempts de doubles liaisons éthyléniques polymérisables,
- de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le le bisphénol A et le bisphénol B, et/ou de dimères diols issus de la réduction des dimères d'acides gras tels que définis précédemment, et
- d'un ou de plusieurs diacides carboxyliques ou leurs anhydrides comportant au moins une double liaison éthylénique polymérisable et ayant de 3 à 50 atomes de carbone, de préférence de 3 à 20 et mieux de 3 à 10 atomes de carbone, tels que l'acide maléique, l'acide fumarique ou l'acide itaconique.

b) les polyesters à groupes (méth)acrylate latéraux et/ou terminaux :

Il s'agit d'un groupe de polymères de type polyester obtenus par polycondensation d'un mélange :

- de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturation éthyléniques tels que les dimères des acides oléique ou linoléique décrits dans la demande EP-A-959 066 (paragraphe [0021]) commercialisés sous les dénominations Pripol® par la société Unichema ou Empol® par la société Henkel, tous ces diacides devant être exempts de doubles liaisons éthyléniques polymérisables,
- de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le bisphénol A et le bisphénol B, et
- d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate

de 2-hydroxypropyle et le méthacrylate de glycérol.

Ces polyesters différent de ceux décrits ci-dessus sous le point a) par le fait que les doubles liaisons éthyléniques ne sont pas situées dans la chaîne principale mais sur des groupes latéraux ou à l'extrémité des chaînes. Ces doubles liaisons éthyléniques sont celles des groupes (méth)acrylate présents dans le polymère.
De tels polyesters sont commercialisés par exemple par la société UCB sous les dénominations EBECRYL® (EBECRYL® 450 : masse molaire 1600, en moyenne 6 fonctions acrylate par molécule, EBECRYL® 652 : masse molaire 1500, en moyenne 6 fonctions acrylate par molécule, EBECRYL® 800 : masse molaire 780, en moyenne 4 fonctions acrylate par molécule, EBECRYL® 810 : masse molaire 1000, en moyenne 4 fonctions acrylate par molécule, EBECRYL® 50 000 : masse molaire 1500, en moyenne 6 fonctions acrylate par molécule).

c) les polyuréthannes et/ou polyurées à groupes (méth)acrylate, obtenus par polycondensation :

- de diisocyanates, triisocyanates et/ou polyisocyanates aliphatiques cycloaliphatiques et/ou aromatiques ayant notamment de 4 à 50, de préférence de 4 à 30 atomes de carbone, tels que l'hexaméthylènediisocyanate, l'isophoronediisocyanate, le toluènediisocyanate, le diphénylméthanediisocyanate ou les isocyanurates de formule :

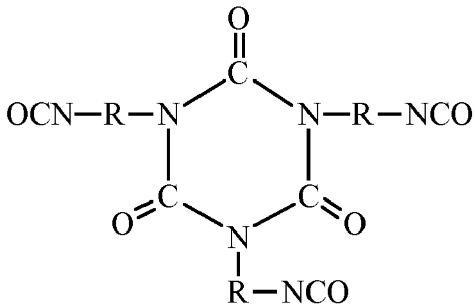

résultant de la trimérisation de 3 molécules de diisocyanates OCN-R-CNO, où R est un radical hydrocarboné linéaire, ramifié ou cyclique comportant de 2 à 30 atomes de carbone ;
- de polyols, notamment de diols, exempts d'insaturations éthyléniques polymérisables, tels que le 1,4-butanediol, l'éthylèneglycol ou le triméthylolpropane, et/ou de polyamines, notamment de diamines, aliphatiques, cycloaliphatiques et/ou aromatiques ayant notamment de 3 à 50 atomes de carbone, telles que l'éthylènediamine ou l'hexaméthylènediamine, et
- d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.

De tels polyuréthannes/polyurées à groupes acrylates sont commercialisés par exemple sous la dénomination SR 368 (tris(2-hydroxyéthyl)isocyanurate-triacrylate) ou CRAYNOR® 435 par la société CRAY VALLEY, ou sous la dénomination EBECRYL® par la société UCB (EBECRYL® 210 : masse molaire 1500, 2 fonctions acrylate par molécule, EBECRYL® 230 : masse molaire 5000, 2 fonctions acrylate par molécule, EBECRYL® 270 : masse molaire 1500, 2 fonctions acrylate par molécule, EBECRYL® 8402 : masse molaire 1000, 2 fonctions acrylate par molécule, EBECRYL® 8804 : masse molaire 1300, 2 fonctions acrylate par molécule, EBECRYL® 220 : masse molaire 1000, 6 fonctions acrylate par molécule, EBECRYL® 2220 : masse molaire 1200, 6 fonctions acrylate par molécule, EBECRYL® 1290 : masse molaire 1000, 6 fonctions acrylate par molécule, EBECRYL® 800 : masse molaire 800, 6 fonctions acrylate par molécule).
On peut également citer les polyuréthannes aliphatiques diacrylate hydrosolubles commercialisés sous les dénominations EBECRYL® 2000, EBECRYL® 2001 et EBECRYL® 2002, et les polyuréthannes diacrylate en dispersion aqueuse commercialisés sous les dénominations commerciales IRR® 390, IRR® 400, IRR® 422 IRR® 424 par la société UCB.

d) les polyéthers à groupes (méth)acrylate obtenus par estérification, par l'acide (méth)acrylique, des groupes hydroxyle terminaux d'homopolymères ou de copolymères d'alkylèneglycols en $C_{1-4}$, tels que le polyéthylèneglycol, le polypropylèneglycol, les copolymères d'oxyde d'éthylène et d'oxyde de propylène ayant de préférence une masse moléculaire moyenne en poids inférieure à 10 000, le triméthylolpropane polyéthoxylé ou polypropoxylé.

Des polyoxyéthylènes-di(méth)acrylate de masse molaire appropriée sont commercialisés par exemple sous les dénominations SR 259, SR 344, SR 610, SR 210, SR 603 et SR 252 par la société CRAY VALLEY ou sous la dénomination EBECRYL® 11 par UCB. Des triacrylates de triméthylolpropane polyéthoxylé sont commercialisés par exemple sous les dénominations SR 454, SR 498, SR 502, SR 9035, SR 415 par la société CRAY VALLEY ou sous la dénomination EBECRYL® 160 par la société UCB. Des triacrylates de triméthylolpropane polypropoxylé sont commercialisés par exemple sous les dénominations SR 492 et SR 501 par la société CRAY VALLEY.

e) les époxyacrylates obtenus par réaction entre

- au moins un diépoxyde choisi par exemple parmi :

  (i) l'éther diglycidylique de bisphénol A,
  (ii) une résine diépoxy résultant de la réaction entre l'éther diglycidylique de bisphénol A et l'épichlorhydrine,
  (iii) une résine époxyester à extrémités $\alpha,\omega$-diépoxy résultant de la condensation d'un diacide carboxylique ayant de 3 à 50 atomes de carbone avec un excès stoechiométrique de (i) et/ou (ii),
  (iv) une résine époxyéther à extrémités $\alpha,\omega$-diépoxy résultant de la condensation d'un diol ayant de 3 à 50 atomes de carbone avec un excès stoechiométrique de (i) et/ou (ii),
  (v) les huiles naturelles ou synthétiques portant au moins 2 groupes époxyde, telles que l'huile de soja époxydée, l'huile de lin époxydée et l'huile de vernonia époxydée,
  (vi) un polycondensat phénol-formaldéhyde (résine Novolac®), dont les extrémités et/ou les groupes latéraux ont été époxydés,

  et
- un ou plusieurs acides carboxyliques ou polyacides carboxyliques comportant au moins une double liaison éthylénique en $\alpha,\beta$ du groupe carboxylique comme l'acide (méth)acrylique ou l'acide crotonique ou les esters d'acide (méth)acrylique et d'un diol ou polyol ayant de 2 à 20 atomes de carbone, de préférence de 2 à 6 atomes de carbone tels que le (méth)acrylate de 2-hydroxyéthyle.

De tels polymères sont commercialisés par exemple sous les dénominations SR 349, SR 601, CD 541, SR 602, SR 9036, SR 348, CD 540, SR 480, CD 9038 par la société CRAY VALLEY, sous les dénominations EBECRYL® 600 et EBECRYL® 609, EBECRYL® 150, EBECRYL® 860, EBECRYL® 3702 par la société UCB et sous les dénominations PHOTOMER® 3005 et PHOTOMER® 3082 par la société HENKEL.

f) les poly(méth)acrylates d'(alkyle en $C_{1-50}$), ledit alkyle étant linéaire, ramifié ou cyclique, comportant au moins deux fonctions à double liaison éthylénique portées par les chaînes hydrocarbonées latérales et/ou terminales. De tels copolymères sont commercialisés par exemple sous les dénominations IRR® 375, OTA® 480 et EBECRYL® 2047 par la société UCB.

g) les polyoléfines telles que le polybutène, le polyisobutylène,

h) les perfluoropolyéthers à groupes acrylate obtenus par estérification, par exemple par l'acide (méth)acrylique, de perfluoropolyéthers portant des groupes hydroxyle latéraux et/ou terminaux.
De tels perfluoropolyéthers $\alpha,\omega$-diols sont décrits notamment dans EP-A-1057849 et sont commercialisés par la société AUSIMONT sous la dénomination FOMBLIN® Z DIOL.

i) les dendrimères et polymères hyperramifiés portant des groupes terminaux (méth)acrylate ou (méth)acrylamide obtenus respectivement par estérification ou amidification de dendrimères et de polymères hyperramifiés à fonctions terminales hydroxyle ou amino, par de l'acide (méth)acrylique.

Les dendrimères (du grec dendron = arbre) sont des molécules polymères "arborescentes", c'est-à-dire très ramifiées inventées par D. A. Tomalia et son équipe au début des années 90 (Donald A. Tomalia et al., Angewandte Chemie, Int. Engl. Ed., vol. 29, n° 2, pages 138 - 175). Il s'agit de structures construites autour d'un motif central généralement polyvalent. Autour de ce motif central, sont enchaînés, selon une structure parfaitement déterminée, des motifs ramifiés d'allongement de chaîne donnant ainsi naissance à des macromolécules symétriques, monodispersées ayant une structure chimique et stéréochimique bien définie. Des dendrimères de type polyamidoamine sont commercialisés par exemple sous la dénomination STARBUST® par la société DENDRITECH.
Les polymères hyperramifiés sont des polycondensats, généralement de type polyester, polyamide ou polyéthylèneamine, obtenus à partir de monomères multifonctionnels, qui ont une structure arborescente similaire à celle des

dendrimères mais beaucoup moins régulière que celle-ci (voir par exemple WO-A-93/17060 et WO 96/12754).

La société PERSTORP commercialise sous la dénomination BOLTORN® des polyesters hyperramifiés. On trouvera sous la dénomination COMBURST® de la société DENDRITECH des polyéthylèneamines hyperramifiées. Des poly(esteramide) hyperramifiés à extrémités hydroxyle sont commercialisés par la société DSM sous la dénomination HYBRANE®.

Ces dendrimères et polymères hyperramifiés estérifiés ou amidifiés par l'acide acrylique et/ou méthacrylique se distinguent des polymères décrits sous les points a) à h) ci-dessus par le très grand nombre de doubles liaisons éthyléniques présentes. Cette fonctionnalité élevée, le plus souvent supérieure à 5, les rend particulièrement utiles en leur permettant de jouer un rôle de "noeud de réticulation", c'est-à-dire de site de réticulation multiple.

On peut donc utiliser ces polymères dendritiques et hyperramifiés en association avec un ou plusieurs des polymères et/ou oligomères a) à h) ci-dessus.

## 1a - Composés réactifs additionnels

Selon un mode de réalisation, les compositions comprenant le composé X et/ou Y peut comprendre en outre un composé réactif additionnel tels que :

- les particules organiques ou minérales comprenant à leur surface au moins 2 groupements aliphatiques insaturés, on peut citer par exemple les silices traitées en surface par exemple par des composés siliconés à groupements vinyliques tels que par exemple la silice traitée cyclotetraméthyltetravinylsiloxane,
- des composés silazanes tels que l'hexaméthyldisilazane.

## 1b - Catalyseur

La réaction d'hydrosilylation se fait en présence d'un catalyseur qui peut être présent avec l'un ou l'autre des composés X ou Y ou être présent de manière isolée. Par exemple, ce catalyseur peut être présent dans la composition sous une forme encapsulée si les deux composés X et Y, dont il doit provoquer l'interaction, sont présents dans cette même composition sous une forme non encapsulée ou à l'inverse il peut y être présent sous une forme non encapsulée si au moins l'un des composés X et Y est présent dans la composition sous une forme encapsulée. Le catalyseur est de préférence à base de platine ou d'étain.

On peut citer par exemple les catalyseurs à base de platine déposé sur un support de gel de silice ou de poudre de charcoal (charbon), le chlorure de platine, les sels de platine et d'acides chloroplatiniques.

On utilise de préférence les acides chloroplatiniques sous forme hexahydrate ou anhydre, facilement dispersible dans les milieux organosiliconés.

On peut également citer les complexes de platine tels que ceux à base d'acide chloroplatinique hexahydrate et de divinyl tetraméthyldisiloxane.

Le catalyseur peut être présent en une teneur allant de 0,0001% à 20% en poids par rapport au poids total de la composition le comprenant.

Les composés X et/ou Y peuvent être associés à des inhibiteurs ou retardateurs de polymérisation, et plus particulièrement des inhibiteurs du catalyseur. De façon non limitative, on peut citer les polyméthylvinylsiloxanes cycliques, et en particulier le tetravinyl tétraméthyl cyclotetrasiloxane, les alcools acétyléniques, de préférence volatils, tels que le méthylisobutynol.

La présence de sels ioniques, tels que l'acétate de sodium peut avoir une influence dans la vitesse de polymérisation des composés.

A titre d'exemple d'une combinaison de composés X et Y réagissant par hydrosilylation en présence d'un catalyseur, on peut citer les références suivantes proposée par la société Dow Corning : DC 7-9800 Soft Skin Adhesive Parts A & B, ainsi que la combinaison des mélanges A et B suivants préparés par Dow Corning :

**MELANGE A :**

| Ingrédient (Nom INCI) | N°CAS | Teneurs (%) | Fonction |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

**MELANGE B :**

| Ingrédient (Nom INCI) | N°CAS | Teneurs (%) | Fonction |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| Dimethyl, Methylhydrogen Siloxane, trimethylsiloxy-terminaux | 68037-59-2 | 1-10 | Polymère |

De façon avantageuse, les composés X et Y sont choisis parmi les composés siliconés susceptibles de réagir par hydrosilylation en présence d'un catalyseur ; en particulier le composé X est choisi parmi les polyorganosiloxanes comprenant des unités de formule (I) décrits ci-dessus et le composé Y est choisi parmi les organosiloxanes comprenant des unités alkylhydrogènosiloxanes de formule (III) décrits ci-dessus.

Selon un mode de réalisation particulier, le composé X est un polydiméthylsiloxane à groupements vinyliques terminaux, et le composé Y est un polyméthylhydrogénosiloxane.

Selon un mode de réalisation particulier, le catalyseur est mis en oeuvre sous une forme encapsulée avec un composé X tel que défini précédemment, et notamment est encapsulé avec un composé X choisi parmi les polyorganosiloxanes comprenant au moins deux groupements aliphatiques insaturés.

**2/ Composés X et Y susceptibles de réagir par condensation**

Selon un mode de réalisation, l'invention concerne une composition cosmétique destinée au soin et/ou au maquillage de matière(s) kératinique(s) comprenant dans un milieu physiologiquement acceptable au moins un composé X et un composé Y, et éventuellement un catalyseur, avec au moins un des composés X et Y étant un polyorganosiloxane, et lesdits composés X et Y étant susceptibles de réagir ensemble par une réaction de condensation, avec au moins un composé parmi les composés X, Y et le catalyseur lorsqu'il est présent, étant présent dans ladite composition sous une forme encapsulée.

Selon ce mode de réalisation, les composés X et Y sont susceptibles de réagir par condensation, soit en présence d'eau (hydrolyse) par réaction de 2 composés porteurs de groupements alcoxysilanes, soit par condensation dite « directe » par réaction d'un composé porteur de groupement(s) alcoxysilane(s) et d'un composé porteur de groupement(s) silanol(s) ou par réaction de 2 composés porteurs de groupement(s) silanol(s).

Lorsque la condensation se fait en présence d'eau, celle-ci peut être en particulier l'humidité ambiante, l'eau résiduelle de la peau, des lèvres des cils et/ou des ongles, ou l'eau apportée par une source extérieure, par exemple par humidification préalable de la matière kératinique (par exemple par un brumisateur, des larmes naturelles ou artificielles).

Dans ce mode de réaction par condensation, les composés X et Y, identiques ou différents, peuvent donc être choisis parmi les composés siliconés dont la chaîne principale comprend au moins deux groupes alcoxysilane et/ou au moins deux groupes silanol (Si-OH), latéraux et/ou en bout de chaîne.

Selon un mode de réalisation, le composé X et/ou le composé Y est porteur d'au moins un groupe polaire, tel que décrit ci-dessus, susceptible de former au moins une liaison hydrogène avec les matières kératiniques.

Selon un mode de réalisation avantageux, les composés X et/ou Y sont choisis parmi les polyorganosiloxanes comprenant au moins deux groupes alcoxysilane. Par groupe « alcoxysilane », on entend un groupe comprenant au moins une partie -Si-OR, R étant un groupe alkyle comprenant de 1 à 6 atomes de carbone.

Les composés X et Y sont notamment choisis parmi les polyorganosiloxanes comprenant des groupes terminaux alcoxysilanes, plus spécifiquement ceux qui comprennent au moins 2 groupes alcoxysilanes terminaux, de préférence trialcoxysilanes terminaux.

Ces composés X et/ou Y comprennent de préférence de façon majoritaire des unités de formule

$$R^9_sSiO_{(4-s)/2}, \qquad (IV)$$

dans laquelle $R^9$ représente indépendamment un radical choisi parmi les groupes alkyles comprenant de 1 à 6 atomes de carbone, le phényle, les groupes alkyl fluorés, et S est égal à 0, 1, 2 ou 3. De préférence, $R^9$ représente indépendamment un groupe alkyle comprenant de 1 à 6 atomes de carbone. Comme groupe alkyle, on peut citer notamment le méthyle, le propyle, le butyle, l'hexyle et leurs mélanges, de préférence le méthyle ou l'éthyle. Comme groupe fluoroalkyle, on peut citer le 3, 3, 3-trifluoropropyle.

Selon un mode de réalisation particulier, les composés X et Y, identiques ou différents, sont des polyorganosiloxanes comprenant des unités de formule

$(R^9{}_2SiO_2)_f$ - (V)

dans laquelle $R^9$ est tel que décrit ci-dessus, de préférence $R^9$ est un radical méthyle, et f est tel que le polymère présente avantageusement une viscosité à 25 °C allant de 0,5 à 3000 Pa.s, de préférence allant de 5 à 150 Pa.s ; par exemple f peut aller de 2 à 5000, de préférence de 3 à 3000, et préférentiellement_de 5 à 1000.

Ces composés X et Y polyorganosiloxanes comprennent au moins 2 groupes trialcoxysilanes terminaux par molécule de polymère, lesdits groupes ayant la formule suivante :

$- ZSiR^1{}_x(OR)_{3-x}$, (VI)

dans laquelle :

les radicaux R représentent indépendamment un groupe méthyle, éthyle, n-propyle, isopropyle, n-butyle, sec-butyle, isobutyle, de préférence un groupe méthyle ou éthyle,
$R^1$ est un groupe méthyle ou éthyle,
x est égal à 0 ou 1, de préférence x est égal à 0 et
Z est choisi parmi : les groupes hydrocarbonés divalents ne comportant pas d'insaturation éthylénique et comprenant de 1 à 18 atomes de carbone, de préférence de 2 à 18 atomes de carbone (groupes alkylène), les combinaisons de radicaux hydrocarbonés divalents et de segments siloxanes de formule (IX) suivante :

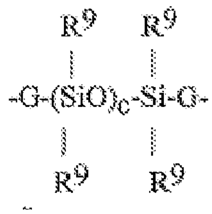

(IX)

$R^9$ étant tel que décrit plus haut, G est un radical hydrocarboné divalent ne comportant pas d'insaturation éthylénique et comprenant de 1 à 18 atomes de carbone, de préférence de 2 à 18 atomes de carbone et c est un entier allant de 1 à 6.
Z et G peuvent être notamment choisis parmi les groupements alkylène tels que le méthylène, l'éthylène, le propylène, le butylène, le pentylène, l'hexylène, les groupements arylène tels que le phenylène

De préférence, Z est un groupe alkylène, et mieux ethylène.

Ces polymères peuvent présenter en moyenne au moins 1,2 groupements terminaux ou chaînes terminales trialcoxysilanes par molécule, et de préférence en moyenne au moins 1,5 groupements terminaux trialcoxysilanes par molécule. Ces polymères pouvant présenter au moins 1,2 groupements terminaux trialcoxysilanes par molécule, certains peuvent comprendre d'autre types de groupes terminaux tels que des groupements terminaux de formule $CH_2$=CH-$SiR^9{}_2$- ou de formule $R^6{}_3$- Si-, dans laquelle $R^9$ est tel que défini plus haut et chaque groupe $R^6$ est indépendamment choisi parmi les groupes $R^9$ ou vinyle. On peut citer comme exemples de tels groupements terminaux les groupes triméthoxysilane, triéthoxysilane, vinyldiméthoxysilane et vinylméthylo xyphénylsilane.

De tels polymères sont notamment décrits dans les documents US 3 175 993, US 4 772 675, US 4 871 827, US 4 888 380, US 4 898 910, US 4 906 719 et US 4 962 174 dont le contenu est incorporé par référence à la présente demande.

On peut citer à titre de composé X et/ou Y en particulier les polyorganosiloxanes choisis parmi les polymères de formule

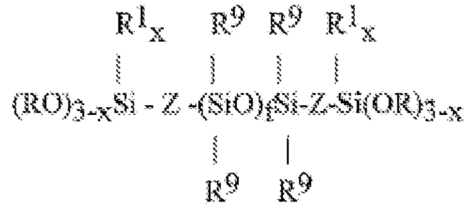

(VII)

dans laquelle R, $R^1$, $R^9$, Z, x et f sont tels que décrits plus haut.

Les composés X et/ou Y peuvent également comprendre un mélanges de polymère de formule (VII) ci-dessus avec des polymères de formule (VIII) suivante :

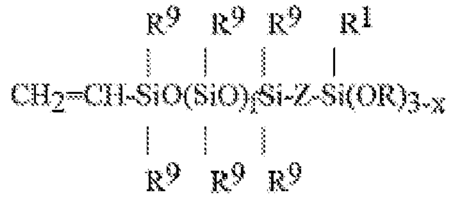

(VIII)

dans laquelle R, $R^1$, $R^9$, Z, x et f sont tels que décrits plus haut.

Lorsque le composé X et/ou Y polyorganosiloxanes à groupe(s) alcoxysilane(s) comprend un tel mélange, les différents polyorganosiloxanes sont présents en des teneurs telles que les chaînes organosilyles terminales représentent moins de 40 %, de préférence moins de 25 % en nombre des chaînes terminales.

[2]Les composés X et/ou Y polyorganosiloxanes particulièrement préférés sont ceux de formule (VII) décrits ci-dessus. De tels composés X et/ou Y sont décrits par exemple dans le document WO 01/96450.

Comme indiqué plus haut, les composés X et Y peuvent être identiques ou différents.

En particulier, les composés X et Y peuvent représenter un mélange de polydiméthylsiloxanes à groupements méthoxysilanes.

Selon une variante l'un des 2 composés réactifs X ou Y, est de nature silicone et l'autre est de nature organique. Par exemple le composé X est choisi parmi les oligomères ou polymères organiques ou les oligomères ou polymères hybrides organique/silicone, lesdits polymères ou oligomères comprenant au moins deux groupements alcoxysilanes et Y est choisi parmi les composés siliconés tels que les polyorganosiloxanes décrits ci-dessus. En particulier, les oligomères ou polymères organiques sont choisis parmi les oligomères ou polymères vinyliques, (méth)acryliques, polyesters, polyamides, polyuréthanes et/ou polyurées, polyéthers, polyoléfines, perfluoropolyéthers, dendrimères et polymères hyper-ramifiés organiques, et leurs mélanges.

Selon un mode de réalisation, le composé X de nature organique ou de nature hybride organique/silicone est porteur d'au moins un groupe polaire, tel que décrit ci-dessus, susceptible de former au moins une liaison hydrogène avec la matière kératinique.

Les polymères organiques de nature vinylique ou (méth)acryliques, porteurs de groupes latéraux alcoxysilanes, pourront en particulier être obtenus par copolymérisation d'au moins un monomère organique vinylique ou (meth)acrylique avec un (méth)acryloxypropyltriméthoxysilane, un vinyl triméthoxysilane, un vinyltriéthoxysilane, un allyltriméthoxysilanes etc..

On peut citer par exemple les polymère (meth)acryliques décrits dans le document de KUSABE.M, Pitture e Verniei - European Coating ; 12-B, pages 43-49, 2005, et notamment les polyacrylates à groupes alcoxysilanes référencés MAX de Kaneka ou ceux décrits dans la publication de PROBSTER, M, Adhesion-Kleben & Dichten, 2004, 481 (1-2), pages 12-14.

Les polymères organiques résultant d'une polycondensation ou d'une polyaddition, tel que les polyesters, polyamides, polyuréthanes et/ou polyurées, polyéthers, et porteurs de groupes alcoysilanes latéraux et/ou terminaux, pourront résulter par exemple de la réaction d'un prépolymère oligomère tel que décrit plus haut avec l'un des co-réactifs silanes suivant porteurs d'au moins un groupe alcoxysilane : aminopropyltriméthoxysilane, aminopropyltriéthoxysilane, aminoéthyl aminopropyl triméthoxysilane, glycidoxypropyltriméthoxysilane, glycidoxypropyltriéthoxysilane, époxycyclohéxyléthyltriméthoxysilane, mercaptopropyltriméthoxysilane.

Des exemples de polyéthers et de polyisobutylènes à groupes alcoxysilanes sont décrits dans la publication de KUSABE.M., Pitture e Verniei - European Coating ; 12-B, pages 43-49, 2005. Comme exemple de polyuréthanes à

groupes alcoxysilanes terminaux , on peut citer ceux décrits dans le document PROBSTER, M., Adhesion-Kleben & Dichten, 2004, 481 (1-2), pages 12-14 ou encore ceux décrits dans le document LANDON, S., Pitture e Verniei vol. 73, N° 11 , pages 18-24, 1997 ou dans le document HUANG, Mowo, Pitture e Verniei vol. 5, 2000, pages 61-67, on peut notamment citer les polyuréthanes à groupes alcoxysilanes de OSI-WITCO-GE.

A titre de composés X et/ou Y polyorganosiloxane, on peut citer les résines de type MQ ou MT portant elle-même des extrémités alcoxysilanes et/ou silanols comme par exemple les résines poly(isobutylsilsesquioxane) fonctionnalisées par des groupes silanols proposées sous la référence SST-S7C41 (3 groupes Si-OH) par la société Gelest.

## 2a Composé réactif additionnel

Selon un mode de réalisation, le composé X et/ou Y peut être associé en outre un composé réactif additionnel comprenant au moins deux groupes alcoxysilane ou silanol.

On peut citer par exemple :

- une ou des particules organiques ou minérales comprenant à leur surface des groupement alcoxysilanes et/ou silanols, par exemple des charges traitées en surface par de tels groupes.

## 2b Catalyseur

La réaction de condensation peut se faire en présence d'un catalyseur à base de métal qui peut être présent avec l'un ou l'autre des composés X ou Y ou être présent de manière isolée. Par exemple, ce catalyseur peut être présent dans la composition sous une forme encapsulée si les deux composés X et Y, dont il doit provoquer l'interaction, sont présents dans cette même composition sous une forme non encapsulée ou à l'inverse il peut y être présent sous une forme non encapsulée si au moins l'un des composés X et Y est présent dans la composition sous une forme encapsulée. Le catalyseur utile dans ce type de réaction est de préférence un catalyseur à base de titane.

On peut citer notamment les catalyseurs à base de tetraalcoxytitane de formule

$$Ti(OR^2)_y(OR^3)_{4-y},$$

dans laquelle $R^2$ est choisi parmi les radicaux alkyle tertiaires tels que le tert butyle, le tert amyle et le 2,4-diméthyl-3-pentyl ; $R^3$ représente un radical alkyle comprenant de 1 à 6 atomes de carbone, de préférence un groupe methyle ethyle, n-propyle, isopropyle, n-butyle, sec-butyle, hexyle et y est un nombre allant de 3 à 4, mieux de 3,4 à 4.

Le catalyseur peut être présent en une teneur allant 0,0001 % à 20 % en poids par rapport au poids total de la composition le contenant.

## 2c Diluant

Les compositions comprenant X et/ou Y peuvent comprendre en outre une huile siliconée volatile (ou diluant) destinée à faire diminuer la viscosité de la composition. Cette huile peut être choisie parmi les silicones linéaires à chaîne courte telles que l'hexamethyldisiloxane, l'octamethyltrisiloxane, les silicones cycliques telles que l'octamethylcyclotetrasiloxane, la decamethylclopentasiloxane et leurs mélanges.

Cette huile siliconée peut représenter de 5 à 95 %, de préférence de 10 à 80 % en poids par rapport au poids de chaque composition.

A titre d'exemple d'une combinaison de composés X et Y porteurs de groupements alcoxysilanes et réagissant par condensation, on peut citer la combinaison des mélanges A' et B' suivants préparés par la société Dow Corning :

**Mélange A' :**

| Ingrédient (Nom INCI) | N°CAS | Teneurs (%) | Fonction |
|---|---|---|---|
| Bis-Trimethoxysiloxyethyl Tetramethyldisilo xyethyl Dimethicone (1) | PMN87176 | 25-45 | Polymère |
| Silica Silylate | 68909-20-6 | 5-20 | Charge |
| Disiloxane | 107-46-0 | 30-70 | Solvant |

**Mélange B' :**

| Ingrédient (Nom INCI) | N°CAS | Teneurs (%) | Fonction |
|---|---|---|---|
| Disiloxane | 107-46-0 | 80-99 | Solvant |
| Tetra T Butyl Titanate | - | 1-20 | Catalyseur |

Il est à noter que les composés X et Y, identiques, sont réunis dans le mélange A' (cf. (1)).

**3/ Réticulation en présence de peroxyde :**

Selon un mode de réalisation, l'invention concerne une composition cosmétique destinée au soin et/ou au maquillage de matière(s) kératinique(s) comprenant dans un milieu physiologiquement acceptable au moins un composé X, un composé Y, et un peroxyde, avec au moins un des composés X ou Y étant un polyorganosiloxane, et lesdits composés X et Y étant susceptibles de réagir ensemble par une réaction de réticulation en présence d'un peroxyde, avec au moins un composé parmi les composés X, Y et le peroxyde étant présent dans ladite composition sous une forme encapsulée.

Cette réaction se fait de préférence par chauffage à une température supérieure ou égale à 50 °C, de préférence supérieure ou égale à 80 °C, allant jusqu'à 120 °C.

Les composés X et Y, identiques ou différents, comprennent dans ce cas au moins deux groupements latéraux $-CH_3$ et/ou au moins deux chaînes latérales portant un groupement $-CH_3$.

Les composés X et Y sont de préférence siliconés et peuvent être choisis par exemple parmi les polydimethylsiloxanes linéaires non volatiles de haut poids moléculaire, ayant un degré de polymérisation supérieur à 6 présentant au moins deux groupements latéraux -CH3 reliés à l'atome de silicium et/ou au moins deux chaînes latérales portant un groupement -CH3. On peut citer par exemple les polymères décrits dans le Catalogue « Reactive Silicones » de la société Gelest Inc., Edition 2004, page 6, et notamment les copolymères (aussi appelés gommes) de vinylmethylsiloxane-dimethylsiloxane de poids moléculaires allant de 500 000 à 900 000 et notamment de viscosité supérieure à 2 000 000 cSt.

A titre de peroxydes utilisables dans le cadre de l'invention, on peut citer le peroxyde de benzoyle, le peroxyde de 2,4-dichlorobenzoyle et leurs mélanges.

Selon un mode de réalisation, la réaction d'hydrosilylation en présence d'un catalyseur ou la réaction de condensation, ou bien encore la réaction de réticulation en présence d'un peroxyde, entre les composés X et Y est accélérée par un apport de chaleur en élevant par exemple la température du système entre 25 °C et 180 °C. Le système réagira notamment sur la matière kératiniques.

D'une façon générale, quel que soit le type de réaction par laquelle les composés X et Y réagissent ensemble, le pourcentage molaire de X par rapport à l'ensemble des composés X et Y, c'est-à-dire le ratio X/(X+Y) x 100, peut varier de 5 % à 95 %, de préférence de 10 % à 90 %, mieux encore de 20 % à 80 %.

De même, le pourcentage molaire de Y par rapport à l'ensemble des composés X et Y, c'est-à-dire le ratio Y/(X+Y) x 100, peut varier de 5 % à 95 %, de préférence de 10 % à 90 %, mieux encore de 20 % à 80 %.

Le composé X peut présenter une masse moléculaire moyenne en poids (Mw) allant de 150 à 1 000 000, de préférence de 200 à 800 000, de préférence encore de 200 à 250 000.

Le composé Y peut présenter une masse moléculaire moyenne en poids (Mw) allant de 200 à 1 000 000, de préférence de 300 à 800 000, de préférence encore de 500 à 250 000.

Le composé X peut représenter de 0,1 % à 95 % en poids par rapport au poids total de la composition le contenant de préférence de 1 % à 90 % et mieux de 5 % à 80 %.

Le composé Y peut représenter de 0,1 % à 95 % en poids par rapport au poids total de la composition le contenant, de préférence de 1 % à 90 % et mieux de 5 % à 80 %.

Le ratio entre les composés X et Y peut être varié de manière à moduler la vitesse de réaction et donc la vitesse de formation du film ou encore de manière à adapter les propriétés du film formé (par exemple ses propriétés adhésives) selon l'application recherchée.

En particulier, les composés X et Y peuvent être présents en un ratio X/Y molaire allant de 0,05 à 20 et mieux de 0,1 à 10.

Les composés X et Y peuvent être associés à une charge. Ainsi, la composition selon l'invention peut comprendre une charge choisie parmi la silice ou la silice traitée en surface.

**MILIEU PHYSIOLOGIQUEMENT ACCEPTABLE**

Comme précisé précédemment, une composition conforme à l'invention comprend un milieu physiologiquement acceptable.

Par « milieu physiologiquement acceptable », on entend désigner un milieu convenant particulièrement à l'application d'une composition selon l'invention sur une matière kératinique comme la peau ou les lèvres.. Le milieu physiologiquement acceptable est généralement adapté à la nature du support sur lequel doit être appliquée la composition, ainsi qu'à l'aspect sous lequel la composition est destinée à être conditionnée.

Le milieu physiologiquement acceptable peut comprendre une phase aqueuse, pouvant comprendre essentiellement de l'eau.

Il peut également comprendre un mélange d'eau et de solvant miscible à l'eau (miscibilité dans l'eau supérieure à 50 % en poids à 25 °C) comme un, ou un mélange de monoalcool(s) inférieur(s) ayant de 1 à 5 atomes de carbone tel(s) que l'éthanol, l'isopropanol, les glycols ayant de 2 à 8 atomes de carbone tels que le porpylène glycol, l'éthylène glycol, le 1,3-butylène glycol, le dipropylène glycol, les cétones en $C_3$-$C_4$, les aldéhydes en $C_2$-$C_4$ et leurs mélanges.

La phase aqueuse (eau et éventuellement le solvant miscible à l'eau) peut être présente, en une teneur allant de 5 à 95 % en poids par rapport au poids total de la composition, de préférence de 10 % à 85 % en poids et mieux de 2 à 80 % en poids.

Le milieu physiologiquement acceptable peut également comprendre une phase grasse liquide, comprenant une ou plusieurs huiles volatiles ou non, et/ou une phase grasse solide comprenant une ou plusieurs cires et/ou composés pâteux, et leur mélange.

Par phase grasse liquide, au sens de la demande, on entend une phase grasse liquide à température ambiante (25°C) et pression atmosphérique (760 mm de Hg), composée d'un ou plusieurs corps gras non aqueux liquides à température ambiante, appelés aussi huiles ou solvants organiques.

L'huile peut être choisie parmi les huiles volatiles et/ou les huile non volatiles, et leurs mélanges.

La ou les huiles peuvent être présentes en une teneur allant de 1 % à 90 % en poids, de préférence de 5 % à 50 % en poids par rapport au poids total de la composition.

Ces huiles peuvent être des huiles hydrocarbonées, des huiles siliconées, des huiles fluorées, ou leurs mélanges.

On entend par « huile hydrocarbonée », une huile contenant principalement des atomes d'hydrogène et de carbone et éventuellement des atomes d'oxygène, d'azote, de soufre, de phosphore. Les huiles hydrocarbonées peuvent être choisies parmi les huiles hydrocarbonées ayant de 8 à 16 atomes de carbones, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars®
ou de Permetyls®, les esters ramifiés en $C_8$-$C_{16}$ le néopentanoate d'iso-hexyle, et leurs mélanges.

Comme huile hydrocarbonée on peut également citer :

les huiles hydrocarbonées d'origine végétale telles que les triesters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,

- les éthers de synthèse ayant de 10 à 40 atomes de carbone ;
- les huiles hydrocarbonées apolaires comme le squalène, les hydrocarbures linéaires ou ramifiés tels que les huiles de paraffine, de vaseline et de naphtalène, le polyisobutène hydrogéné ou partiellement hydrogéné, l'isoeicosane, le squalane, les copolymères décène/butène, les copolymères polybutène/polyisobutène notamment l'Indopol L-14, les polydécènes tel que le PURESYN 10, et leurs mélanges
- les esters de synthèse comme les huiles de formule $R_1COOR_2$ dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que $R_1 + R_2$ soit ≥ 10, comme par exemple l'huile de Purcellin (octanoate de cétostéaryle), le myristate d'isopropyle, le palmitate d'isopropyle, le benzoate d'alcool en $C_{12}$ à $C_{15}$, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;

- et leurs mélanges.

Comme huile de silicone utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

Les huiles de silicone peuvent également être :

- les polydiméthylsiloxanes (PDMS),
- les polydiméthylsiloxanes comportant des groupements alkyle ou alcoxy, pendant et/ou en bout de chaîne siliconée, groupements ayant chacun de 3 à 40 atomes de carbone,
- les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;
- et leurs mélanges.

Les compositions selon l'invention peuvent également comprendre au moins un corps gras solides à température ambiante notamment choisi parmi les cires, les corps gras pâteux et leurs mélanges. Ces corps gras peuvent être d'origine animale, végétale, minérale ou synthétique.

Les compositions selon l'invention peuvent également contenir des ingrédients couramment utilisés en cosmétique, tels que les vitamines, les épaississants, les gélifiants, les oligo-éléments, les adoucissants, les séquestrants, les parfums, les agents alcalinisants ou acidifiants, les conservateurs, les parfums, les filtres solaires, les tensioactifs, les anti-oxydants, les fibres, les charges, les matières colorantes, les polymères filmogènes, les cires, les actifs cosmétiques comme les actifs bactéricides ou anti-transpirants, les neutralisants, les émollients, les hydratants, et leurs mélanges.

Les compositions selon l'invention peuvent contenir des agents tensioactifs émulsionnants présents notamment en une proportion allant de 0,1 à 30 % en poids par rapport au poids total de la composition, mieux de 1 à 15 % et mieux de 2 à 10 %.

Ces agents tensioactifs peuvent être choisis parmi des agents tensioactifs anioniques, non ioniques, amphotères ou zwitterioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs non ioniques de l'invention sont préférentiellement choisis parmi :

1/ les tensioactifs siliconés,
2/ les tensioactifs liquides à température inférieure ou égale à 45 °C, choisis parmi les esters d'au moins un polyol d'au moins un acide gras comportant au moins une chaîne alkyle en $C_8$-$C_{22}$, saturée ou non saturée, linéaire ou ramifiée, et notamment insaturée ou ramifiée, le polyol étant choisi dans le groupe formé par le polyéthylèneglycol comportant de 1 à 60 unités d'oxyde d'éthylène, le sorbitan, le glycérol pouvant comporter de 2 à 30 unités d'oxyde d'éthylène, les polyglycérols comportant de 2 à 15 unités de glycérol,
3/ les esters d'acide gras et de sucre et les éthers d'alcool gras et de sucre,
4/ les tensioactifs solides à une température égale à 45 °C, choisis parmi les esters gras de glycérol, les esters gras de sorbitan et les esters gras de sorbitan oxyéthylénés, les éthers gras éthoxylés et les esters gras éthoxylés,
5/ Les copolymères blocs d'oxyde d'éthylène (A) et d'oxyde de propylène (B), et les mélanges de ces tensioactifs.

1/ Les tensioactifs siliconés utilisables selon l'invention sont des composés siliconés comportant au moins une chaîne oxyéthylénée $-OCH_2CH_2-$ et/ou oxypropylénée - $OCH_2CH_2CH_2-$. Comme tensioactifs siliconés pouvant être utilisés selon la présente invention, on peut citer ceux décrits dans les documents US-A-5,364,633 et US-A-5,411,744.

De préférence, le tensioactif siliconé utilisé selon la présente invention est un composé de formule (II) :

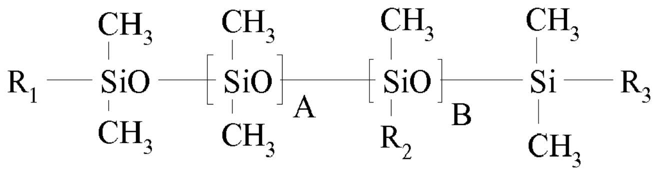

(II)

dans laquelle :

$R_1$, $R_2$, $R_3$, indépendamment les uns des autres, représentent un radical alkyle en $C_1$-$C_6$ ou un radical $-(CH_2)_x-(OCH_2CH_2)_y-(OCH_2CH_2CH_2)_z-OR_4$, au moins un radical $R_1$, $R_2$ ou $R_3$ n'étant pas un radical alkyle ;
$R_4$ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

Selon un mode de réalisation préféré de l'invention, dans le composé de formule (II), le radical alkyle est un radical méthyle, x est un nombre entier allant de 2 à 6 et y est un nombre entier allant de 4 à 30.
On peut citer, à titre d'exemple de tensioactifs siliconés de formule (II), les composés de formule (III) :

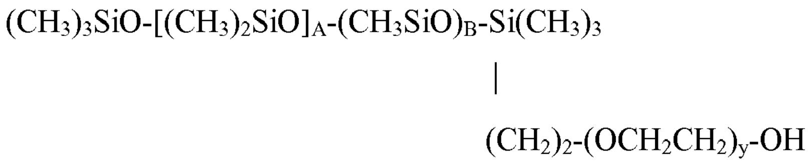

(III)

dans laquelle A est un nombre entier allant de 20 à 105, B est un nombre entier allant de 2 à 10 et y est un nombre entier allant de 10 à 20.
On peut également citer à titre d'exemple de tensioactifs siliconés de formule (II), les composés de formule (IV) :

$$H-(OCH_2CH_2)_y-(CH_2)_3-[(CH_3)_2SiO]_{A'}-(CH_2)_3-(OCH_2CH_2)_y-OH \qquad (IV)$$

dans laquelle A' et y sont des nombres entiers allant de 10 à 20.
On peut utiliser notamment comme tensioactifs siliconés, ceux commercialisés par la société Dow Corning sous les dénominations DC 5329, DC 7439-146, DC 2-5695 et Q4-3667. Les composés DC 5329, DC 7439-146, DC 2-5695 sont des composés de formule (III) où respectivement A est 22, B est 2 et y est 12 ; A est 103, B est 10 et y est 12 ; A est 27, B est 3 et y est 12.
Le composé Q4-3667 est un composé de formule (IV) où A est 15 et y est 13.
2/ Les tensioactifs liquides à température inférieure ou égale à 45 °C peuvent être notamment choisis parmi :

- l'isostéarate de polyéthylèneglycol de poids molaire 400 (nom CTFA : PEG-8 Isostearate), vendu sous la dénomination Prisorine 3644 par la société UNICHEMA ;
- l'isostéarate de diglycéryle, vendu par la société SOLVAY ;
- le laurate de polyglycérol comportant 2 unités de glycérol (polyglyceryl-2 laurate), vendu sous la dénomination Diglycerin-monolaurate par la société SOLVAY ;
- l'oléate de sorbitan, vendu sous la dénomination SPAN 80 par la société ICI,
- l'isostéarate de sorbitan, vendu sous la dénomination NIKKOL SI 10R par la société NIKKO ;
- le cocoate d'$\alpha$-butylglucoside ou le caprate d'$\alpha$-butylglucoside commercialisés par la société ULICE.

3/ Les esters d'acide gras et de sucre, utilisables comme lipides amphiphiles non ioniques dans la nanoémulsion selon l'invention sont de préférence solides à une température inférieure ou égale à 45 °C et peuvent être choisis notamment dans le groupe comprenant les esters ou les mélanges d'esters d'acide gras en $C_8$-$C_{22}$ et de sucrose,

de maltose, de glucose ou de fructose, et les esters ou les mélanges d'esters d'acide gras en $C_{14}$-$C_{22}$ et de méthylglucose.

Les acides gras en $C_8$-$C_{22}$ ou en $C_{14}$-$C_{22}$ formant le motif gras des esters utilisables dans la nanoémulsion de l'invention comportent une chaîne alkyle linéaire saturée ou non saturée, ayant respectivement de 8 à 22 ou de 14 à 22 atomes de carbone. Le motif gras des esters peut être notamment choisi parmi les stéarates, béhénates, arachidonates, palmitates, myristates, laurates, caprates et leurs mélanges. On utilise de préférence des stéarates.

On peut citer, à titre d'exemple d'esters ou de mélanges d'esters d'acide gras et de sucrose, de maltose, de glucose ou de fructose, le monostéarate de sucrose, le distéarate de sucrose, le tristéarate de sucrose et leurs mélanges, tels que les produits commercialisés par la société Croda sous la dénomination Crodesta F50, F70, F110, F160 ayant respectivement un HLB (Hydrophilic Lipophilic Balance) de 5, 7, 11 et 16 ; et à titre d'exemple d'esters ou de mélanges d'esters d'acide gras et de méthylglucose, le distéarate de méthyl glucose et de polyglycérol-3, vendu par la société Goldschmidt sous la dénomination de Tego-care 450. On peut citer aussi les monoesters de glucose ou de maltose tels que l'o-hexadécanoyle-6-D-glucoside de méthyle et l'o-hexadécanoyle-6-D-maltoside de méthyle.

Les éthers d'alcool gras et de sucre, utilisables comme lipides amphiphiles non ioniques dans la nanoémulsion selon l'invention sont solides à une température inférieure ou égale à 45 °C et peuvent être choisis notamment dans le groupe comprenant les éthers ou mélanges d'éthers d'alcool gras en $C_8$-$C_{22}$ et de glucose, de maltose, de sucrose ou de fructose et les éthers ou mélanges d'éthers d'alcool gras en $C_{14}$-$C_{22}$ et de méthylglucose. Ce sont notamment des alkylpolyglucosides.

Les alcools gras en $C_8$-$C_{22}$ ou en $C_{14}$-$C_{22}$ formant le motif gras des éthers utilisables dans la nanoémulsion de l'invention comportent une chaîne alkyle linéaire saturée ou non saturée, ayant respectivement de 8 à 22 ou de 14 à 22 atomes de carbone. Le motif gras des éthers peut être notamment choisi parmi les motifs décyle, cétyle, béhényle, arachidyle, stéaryle, palmityle, myristyle, lauryle, capryle, hexadécanoyle, et leurs mélanges tels que cétéaryle.

A titre d'exemple d'éthers d'alcool gras et de sucre, on peut citer les alkylpolyglucosides tels que le décylglucoside et le laurylglucoside commercialisés par exemple par la société Henkel sous les dénominations respectives de Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tego-care CG90 par la société Goldschmidt et sous la dénomination Emulgade KE3302 par la société Henkel, ainsi que l'arachidylglucoside, par exemple sous la forme du mélange d'alcools arachidique et béhénique et d'arachidylglucoside, commercialisé sous la dénomination Montanov 202 par la société Seppic.

On utilise plus particulièrement comme tensioactif non ionique de ce type, le monostéarate de sucrose, le distéarate de sucrose, le tristéarate de sucrose et leurs mélanges, le distéarate de méthyl glucose et de polyglycérol-3 et les alkylpolyglucosides.

4/ Les esters gras de glycérol, utilisables comme tensioactifs non ioniques solides à une température égale à 45 °C, peuvent être choisis notamment dans le groupe comprenant les esters formés d'au moins un acide comportant une chaîne alkyle linéaire saturée, ayant de 16 à 22 atomes de carbone, et de 1 à 10 motifs glycérol. On peut utiliser un ou plusieurs de ces esters gras de glycérol.

Ces esters peuvent être notamment choisis parmi les stéarates, béhénates, arachidates, palmitates et leurs mélanges. On utilise de préférence des stéarates et palmitates.

On peut citer à titre d'exemple de tensioactif utilisable dans la nanoémulsion de l'invention, les monostéarate, distéarate, tristéarate et pentastéarate de décaglycérol (10 unités de glycérol) (noms CTFA : Polyglyceryl-10 stearate, Polyglyceryl-10 distearate, Polyglyceryl-10 tristearate, Polyglyceryl-10 pentastearate) tels que les produits vendus sous les dénominations respectives Nikkol Decaglyn 1-S, 2-S, 3-S et 5-S par la société Nikko, et le monostéarate de diglycérol (nom CTFA : Polyglyceryl-2 stearate) tel que le produit vendu par la société Nikko sous la dénomination Nikkol DGMS.

Les esters gras de sorbitan, utilisables comme tensioactifs non ioniques solides à une température inférieure ou égale à 45 °C, sont choisis notamment dans le groupe comprenant les esters d'acide gras en $C_{16}$-$C_{22}$ et de sorbitan et les esters d'acide gras en $C_{16}$-$C_{22}$ et de sorbitan oxyéthylénés. Ils sont formés d'au moins un acide gras comportant au moins une chaîne alkyle linéaire saturée, ayant respectivement de 16 à 22 atomes de carbone, et de sorbitol ou de sorbitol éthoxylé. Les esters oxyéthylénés comportent généralement de 1 à 100 unités d'oxyde d'éthylène et de préférence de 2 à 40 unités d'oxyde d'éthylène (OE).

Ces esters peuvent être notamment choisis parmi les stéarates, béhénates, arachidates, palmitates, et leurs mélanges. On utilise de préférence des stéarates et palmitates.

On peut citer à titre d'exemple d'ester gras de sorbitan et d'ester gras de sorbitan oxyéthyléné, utilisable dans la nanoémulsion de l'invention, le monostéarate de sorbitan (nom CTFA : Sorbitan stearate) vendu par la société ICI sous la dénomination Span 60, le monopalmitate de sorbitan (nom CTFA : Sorbitan palmitate) vendu par la société ICI sous la dénomination Span 40, le tristéarate de sorbitan 20 OE (nom CTFA : Polysorbate 65) vendu par la société ICI sous la dénomination Tween 65.

Les éthers gras éthoxylés solides à une température inférieure ou égale à 45 °C sont de préférence des éthers formés de 1 à 100 unités d'oxyde d'éthylène et d'au moins une chaîne d'alcool gras ayant de 16 à 22 atomes de carbone. La chaîne grasse des éthers peut être notamment choisie parmi les motifs béhényle, arachidyle, stéaryle, cétyle, et leurs mélanges tels que cétéaryle. A titre d'exemple d'éthers gras éthoxylés, on peut citer les éthers d'alcool béhénique comprenant 5, 10, 20 et 30 unités d'oxyde d'éthylène (noms CTFA : Beheneth-5, Beheneth-10, Beheneth-20, Beheneth-30), tels que les produits commercialisés sous les dénominations Nikkol BB5, BB10, BB20, BB30 par la société Nikko, et l'éther d'alcool stéarylique comprenant 2 unités d'oxyde d'éthylène (nom CTFA : Steareth-2), tel que le produit commercialisé sous la dénomination Brij 72 par la société ICI.

Les esters gras éthoxylés solides à une température inférieure ou égale à 45 °C sont des esters formés de 1 à 100 unités d'oxyde d'éthylène et d'au moins une chaîne d'acide gras comportant de 16 à 22 atomes de carbone. La chaîne grasse des esters peut être notamment choisie parmi les motifs stéarate, béhénate, arachidate, palmitate, et leurs mélanges. A titre d'exemple d'esters gras éthoxylés, on peut citer l'ester d'acide stéarique comprenant 40 unités d'oxyde d'éthylène, tel que le produit commercialisé sous la dénomination Myrj 52 (nom CTFA : PEG-40 stearate) par la société ICI ainsi que l'ester d'acide béhénique comprenant 8 unités d'oxyde d'éthylène (nom CTFA : PEG-8 behenate), tel que le produit commercialisé sous la dénomination Compritol HD5 ATO par la société Gattefosse.

5/ Les copolymères blocs d'oxyde d'éthylène et d'oxyde de propylène, utilisables comme tensioactifs non ioniques peuvent être choisis notamment parmi les copolymères blocs de formule (V) :

$$HO(C_2H_4O)x(C_3H_6O)y(C_2H_4O)zH \qquad (V)$$

dans laquelle x, y et z sont des nombres entiers tels que x+z va de 2 à 100 et y va de 14 à 60, et leurs mélanges, et plus particulièrement parmi les copolymères blocs de formule (V) ayant un HLB allant de 2 à 16.

Ces copolymères blocs peuvent être notamment choisis parmi les polycondensats tribloc polyéthylène glycol / polypropylène glycol / polyéthylène glycol. vendus sous les dénominations « SYNPERONIC » comme les « SYNPERONIC® PE/L81 » (nom INCI : POLOXAMER 231) de formule (V) avec x=z=6, y=39 (HLB 2) ; « SYNPERONIC® PE/L92 » (nom INCI : POLOXAMER 282) de formule (V) avec x=z=10, y=47 (HLB 6) par la société UNIQEMA.

Comme tensioactifs non ioniques, on peut aussi citer les mélanges de tensioactifs non ioniques décrits dans le document EP-A-705 593 incorporé ici pour référence.

Parmi les tensioactifs non ioniques, on peut utiliser en particulier :

- l'isostéarate de PEG 400 ou PEG-8 Isostéarate (comportant 8 moles d'oxyde d'éthylène),
- l'isostéarate de diglycéryle,
- le monolaurate de polyglycérol comportant 2 unités de glycérol et les stéarates de polyglycérol comportant 10 unités de glycérol,
- l'oléate de sorbitan,
- l'isostéarate de sorbitan,
- et leurs mélanges.

Les tensioactifs anioniques pouvant être utilisés dans les compositions de l'invention sont choisis de préférence parmi :

- les sels alcalins du dicétyl- et du dimyristylphosphate ;
- les sels alcalins du cholestérol sulfate ;
- les sels alcalins du cholestérol phosphate ;
- les lipoaminoacides et leurs sels tels que les acylglutamates mono- et di-sodiques comme le sel disodique de l'acide N-stéaroyl L-glutamique commercialisé sous la dénomination Acylglutamate HS21 par la société AJINOMOTO ;
- les sels de sodium de l'acide phosphatidique ;
- les phospholipides ;
- les dérivés alkylsulfoniques notamment de formule (VI) :

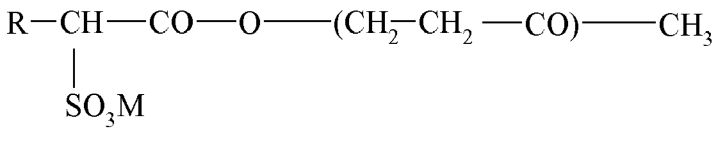

(VI)

dans laquelle R représente des radicaux alkyle en $C_{16}$-$C_{22}$, en particulier les radicaux $C_{16}H_{33}$ et $C_{18}H_{37}$ pris en mélange ou séparément et M est un métal alcalin ou alcalino-terreux tel que le sodium ; et leurs mélanges.

Les tensioactifs cationiques pouvant être utilisés dans les compositions de l'invention sont choisis, de préférence, dans le groupe formé par les sels d'ammonium quaternaire, les amines grasses et leurs sels.

Les sels d'ammonium quaternaires sont par exemple :

- ceux qui présentent la formule générale (VII) suivante :

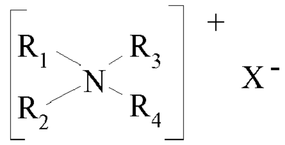

(VII)

dans laquelle les radicaux $R_1$ à $R_4$, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène($C_2$-$C_6$), alkylamide, alkyl($C_{12}$-$C_{22}$)amido alkyle($C_2$-$C_6$), alkyl($C_{12}$-$C_{22}$)acétate, hydroxyalkyle, comportant environ de 1 à 30 atomes de carbone; X est un anion choisi dans le groupe des halogénures, phosphates, acétates, lactates, alkyl($C_2$-$C_6$)sulfates, alkyl-ou-alkylarylsulfonates,

Parmi les sels d'ammonium quaternaire de formule (VII), on préfère, d'une part, les chlorures de tétraalkylammonium comme par exemple les chlorures de dialkyl-diméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthyl-ammonium, de benzyl diméthyl stéaryl ammonium ou encore, d'autre part, le chlorure de stéaramidopropyldiméthyl (myristyl acétate) ammonium commercialisé sous la dénomination «CERAPHYL 70» par la société VAN DYK. Le chlorure de béhényltriméthylammonium est le sel d'ammonium quaternaire le plus particulièrement préféré.

- les sels d'ammonium quaternaire de l'imidazolinium, comme par exemple celui de formule (VIII) suivante :

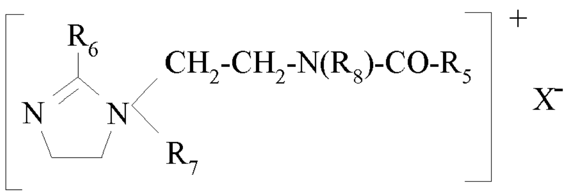

VIII

dans laquelle $R_5$ représente un radical alcényle ou alkyle comportant de 8 à 30 atomes de carbone par exemple dérivés des acides gras du suif, $R_6$ représente un atome d'hydrogène, un radical alkyle en $C_1$-$C_4$ ou un radical alcényle ou alkyle comportant de 8 à 30 atomes de carbone, $R_7$ représente un radical alkyle en $C_1$-$C_4$, $R_8$ représente un atome d'hydrogène, un radical alkyle en $C_1$-$C_4$, X est un anion choisi dans le groupe des halogénures, phosphates, acétates, lactates, alkylsulfates, alkyl-ou-alkylarylsulfonates. De préférence, $R_5$ et $R_6$ désignent un mélange de radicaux alcényle ou alkyle comportant de 12 à 21 atomes de carbone par exemple dérivés des acides gras du suif, $R_7$ désigne méthyle, $R_8$ désigne hydrogène. Un tel produit est par exemple commercialisé sous la dénomination «REWOQUAT W 75» par la société REWO,

- les sels de diammonium quaternaire de formule (IX) :

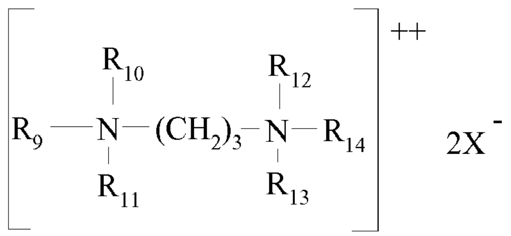

(IX)

dans laquelle $R_9$ désigne un radical aliphatique comportant environ de 16 à 30 atomes de carbone, $R_{10}$, $R_{11}$, $R_{12}$, $R_{13}$ et $R_{14}$ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.

- les sels d'ammonium quaternaire contenant au moins une fonction ester,
  Les sels d'ammonium quaternaire contenant au moins une fonction ester utilisables selon l'invention sont par exemple ceux de formule X suivante :

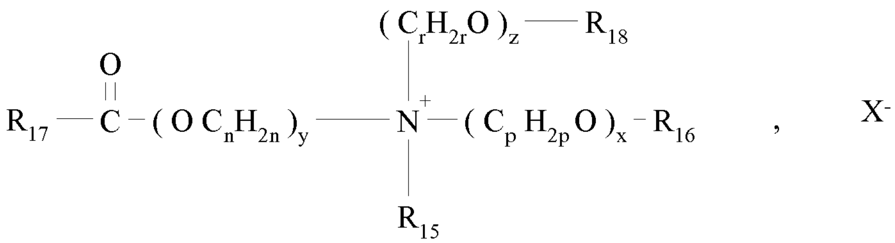

(X)

dans laquelle :

- $R_{15}$ est choisi parmi les radicaux alkyles en $C_1$-$C_6$ et les radicaux hydroxyalkyles ou dihydroxyalkyles en $C_1$-$C_6$ ;
- $R_{16}$ est choisi parmi :
- le radical $R_{19}$

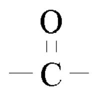

- les radicaux $R_{20}$ hydrocarbonés en $C_1$-$C_{22}$ linéaires ou ramifiés, saturés ou insaturés,
- l'atome d'hydrogène,
- $R_{18}$ est choisi parmi :
- le radical $R_{21}$

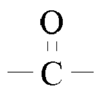

- les radicaux $R_{22}$ hydrocarbonés en $C_1$-$C_6$ linéaires ou ramifiés, saturés ou insaturés,
- l'atome d'hydrogène,
- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;

- X- est un anion simple ou complexe, organique ou inorganique ;

sous réserve que la somme x + y + z vaut de 1 à 15 , que lorsque x vaut 0 alors $R_{16}$ désigne $R_{20}$ et que lorsque z vaut 0 alors $R_{18}$ désigne $R_{22}$.

Les radicaux alkyle $R_{15}$ peuvent être linéaires ou ramifiés et plus particulièrement linéaires.

De préférence $R_{15}$ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque $R_{16}$ est un radical $R_{20}$ hydrocarboné, il peut être long et avoir de 12 à 22 atomes de carbone ou court et avoir de 1 à 3 atomes de carbone.

Lorsque $R_{18}$ est un radical $R_{22}$ hydrocarboné, il a de préférence 1 à 3 atomes de carbone.

Avantageusement, $R_{17}$, $R_{19}$ et $R_{21}$, identiques ou différents, sont choisis parmi les radicaux hydrocarbonés en $C_{11}$-$C_{21}$, linéaires ou ramifiés, saturés ou insaturés, et plus particulièrement parmi les radicaux alkyle et alcényle en $C_{11}$-$C_{21}$, linéaires ou ramifiés, saturés ou insaturés.

De préférence, x et z, identiques ou différents, valent 0 ou 1.

Avantageusement, y est égal à 1.

De préférence, n, p et r, identiques ou différents, valent 2 ou 3 et encore plus particulièrement sont égaux à 2.

Dans la formule X, l'anion X- est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester. L'anion X- est encore plus particulièrement le chlorure ou le méthylsulfate.

On utilise plus particulièrement les sels d'ammonium de formule X dans laquelle :

- R15 désigne un radical méthyle ou éthyle,
- x et y sont égaux à 1 ;
- z est égal à 0 ou 1 ;
- n, p et r sont égaux à 2 ;
- R16 est choisi parmi :
- le radical $R_{19}$

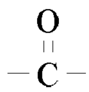

- les radicaux méthyle, éthyle ou hydrocarbonés en $C_{14}$-$C_{22}$
- l'atome d'hydrogène ;
- $R_{18}$ est choisi parmi :
- le radical $R_{21}$

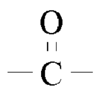

- l'atome d'hydrogène ;

$R_{17}$, $R_{19}$ et $R_{21}$, identiques ou différents, sont choisis parmi les radicaux hydrocarbonés en $C_{13}$-$C_{17}$, linéaires ou ramifiés, saturés ou insaturés et de préférence parmi les radicaux alkyles et alcényle en $C_{13}$-$C_{17}$, linéaires ou ramifiés, saturés ou insaturés.

Avantageusement, les radicaux hydrocarbonés sont linéaires.

On peut citer par exemple comme composés de formule X les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents. Ces produits sont obtenus par exemple par estérification directe de la triéthanolamine, de la triisopropanolamine, d'alkyldiéthanolamine ou d'alkyldiiso-propanolamine éventuellement

oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

Quand elle contient des sels d'ammonium, la composition selon l'invention contient de préférence un mélange de sels de mono, di et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl dihydroxyéthyl méthyl ammonium, 45 à 60 % de méthylsulfate de diacyloxyéthyl hydroxyéthyl méthyl ammonium et 15 à 30 % de méthylsulfate de triacyloxyéthyl méthyl ammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Comme tensioactifs, on peut également utiliser les émulsionnants polyoléfines à partie polaire, qui sont notamment décrites par exemple dans la demande EP-A-1172089.

Les polyoléfines à partie(s) polaire(s) utilisées dans la composition de l'invention sont constituées d'une partie apolaire polyoléfinique et d'au moins une partie polaire. Elles peuvent présenter une structure de type bloc ou peigne.

On peut utiliser des polyoléfines à partie polaire polyoxyéthylène par exemple choisies parmi les polymères diblocs polyisoprène-polyoxyéthylène, les polymères poly(éthylène-co-propylène)-polyoxyéthylène et leurs mélanges. Ces polymères sont décrits dans la publication de Allgaier, Poppe, Willner, Richter (Macromolecules, 1997, vol. 30, p. 1582-1586).

On peut utiliser des polyoléfines à partie polaire acide ou anhydride succinique choisies notamment parmi les dérivés polyoléfines d'acide ou d'anhydride succinique décrits dans les brevets US-A-4,234,435, US-A-4,708,753, US-A-5,129,972, US-A-4,931,110, GB-A-2,156,799, US-A-4,877,756 et US-A-4,919,179.

On peut utiliser les polyoléfines à terminaison succinique modifiée (estérifiée ou amidifiée) et leur procédé de préparation sont décrits en particulier dans le document US-A-4,708,753. On utilise de préférence des polyoléfines à terminaison succinique estérifiée.

Comme polyoléfines à terminaison succinique, on peut citer notamment les polyisobutylènes à terminaison succinique estérifiée, notamment estérifiée par la diéthanolamine, et leurs sels, notamment les sels de diéthanolamine, tels que les produits commercialisés sous les dénominations Lubrizol® 2724, Lubrizol® 2722 et Lubrizol® 5603 par la société Lubrizol.

Un autre exemple de polyoléfine à partie polaire utilisable dans l'invention est le produit de la réaction de l'anhydride maléique avec le polyisobutylène, tel que les produits commercialisés sous les dénominations Glissopal (Glissopal 2300, 1300 et 1000) (nom INCI : polyisobutene) par la société BASF.

La polyoléfine à partie polaire particulièrement préférée est un produit réactionnel de l'anhydride polyisobuténylsuccinique avec de la diéthyléthanolamine, formant ainsi un sel de diéthyléthanolamine de polybutène succinate de 2-(N, N diéthyl)-amino éthyle. Ce produit est vendu par exemple sous la dénomination Lubrizol® 5603 par la société Lubrizol, Ce produit a pour nom INCI : Hydroxyethyldiethonium Polyisobutenyl Triethylaminosuccinate (and) Diethyl ethanolamine.

Une autre polyéoléfine à partie polaire particulièrement préférée est un ester de polyisobuténylsuccinate de diéthanolaminoéthyle et de triéthanolamnine. Ce produit est vendu par exemple sous la dénomination Chemccinate® 2000 par la société Chemron.

Comme polyoléfine à partie polaire, on peut également utiliser un ester de polyisobuténylsuccinate de glycéryle, notamment celui vendu sous la dénomination Chemccinate® 1000 AF par la société Chemron.

Comme tensioactifs, on peut également utiliser les élastomères de silicones émulsionnants tels que ceux vendus sous les dénominations KSG-210, KSG-310, KSG-320, KSG-330, KSG-440, KSG-710, KSG-KSG-830, KSG-840 par la société SHIN-ETSU.

On peut également utiliser les polymères d'acide isophtalique ou d'acide sulfoisophtalique, et en particulier les copolymères de phtalate / sulfoisophtalate / glycol (par exemple diéthylèneglycol / Phtalate / isophtalate / 1,4-cyclohexane-diméthanol) vendus sous les dénominations « Eastman AQ polymer » (AQ35S, AQ38S, AQ55S, AQ48 Ultra) par la société Eastman Chemical. De tels tensioactifs sont décrits dans la demande EP-A-864320.

On peut également utiliser les polymères hydrophobes de l'acide 2-acrylamido-2-méthylpropane-sulfonique (AMPS) tels que le copolymère d'AMPS et de methacrylate d'alcool en C12-C14 éthoxylé (copolymère non réticulé obtenu à partir de Genapol LA-070 et d'AMPS) (nom CTFA : Ammonium Acryloyldimethyltaurate / Laureth-7 Methacrylate Copolymer) commercialisé sous la dénomination ARISTOFLEX LNC par la société Clariant, et le copolymère d'AMPS et de méthacrylate de stéaryle éthoxylé (25 EO) (copolymère réticulé par le trimethylolpropane triacrylate, obtenu à

partir de Genapol T-250 et d'AMPS) (nom CTFA : Ammonium Acryloyldimethyltaurate / Steareth-25 Methacrylate Crosspolymer) commercialisé sous la dénomination ARISTOFLEX HMS par la société Clariant. Ces polymères sont notamment décrits dans la demande EP-A-1661550.

Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée,

Les compositions selon l'invention peu(ven)t se présenter indépendamment sous forme de suspension, de dispersion, de solution, de gel, d'émulsion, notamment émulsion huile-dans-eau (H/E), cire-dans-eau ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de pâte, notamment de pâte souple.

Les compositions selon l'invention ou utilisées pour le procédé selon l'invention peuvent se présenter sous la forme d'une composition de protection, de traitement ou de soin pour le visage, pour les mains, pour les pieds, pour les grands plis anatomiques ou pour le corps (par exemple crèmes de jour, crème de nuit, crème démaquillante, composition anti-solaire, lait corporels de protection ou de soin, laits après-solaire, lotion, gel ou mousse pour le soin de la peau, composition de bronzage artificiel); une composition après-rasage.

Elles peuvent également être utilisées pour le maquillage de la peau, des lèvres, des cils et/ou des ongles selon la nature des ingrédients utilisés.

En particulier, les compositions selon l'invention peu(ven)t se présenter indépendamment sous forme, de fond de teint, de produit pour les lèvres, notamment de rouge à lèvres, de produit anti-cernes, ou contours des yeux, d'eyes liner, de mascara, d'ombre à paupières, de produit de maquillage du corps ou encore un produit de coloration de la peau.

Selon un mode de réalisation, les compositions sont des compositions de revêtement de la peau du corps ou du visage plus particulièrement des compositions de maquillage ou de soin de la peau du corps ou du visage telles que par exemple des fonds de teint ou des compositions de maquillage du corps.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour chaque composition.

L'invention est illustrée plus en détails par les exemples décrits ci-après. Sauf indication contraire, les quantités indiquées sont exprimées en pourcentage massique.

Dans les exemples de compositions décrites ci-après, on utilise, à titre de composés X et Y, la combinaison des mélanges A et B suivants préparés par la société Dow Corning :

**MELANGE A :**

| **Ingrédient (Nom INCI)** | **N°CAS** | **Teneurs (%)** | **Fonction** |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

**MELANGE B :**

| **Ingrédient (Nom INCI)** | **N°CAS** | **Teneurs (%)** | **Fonction** |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| Dimethyl, Methylhydrogen Siloxane, trimethylsiloxy-terminaux | 68037-59-2 | 1-10 | Polymère |

**Exemple 1 :**

Préparation de nanocapsules d'un composé X ou Y conformes à l'invention

| | |
|---|---|
| Phase organique : | |
| Mélange B | 3,62 g |

(suite)

| | |
|---|---|
| Phase organique : | |
| Polycaprolactone (Capa®6100, Solvay) | 0,48 g |
| Diméthicone copolyol (DC SH 3773 M, Dow Corning) | 0,24g |
| Dichlorométhane | 16 ml |
| Phase aqueuse : | |
| Acide N Stéaroyl L glutamique disodique | |
| (Amisoft HS21P d'Ajinomoto) | 0,12 g |
| Eau distillée | 24,14 g |

Une pré-émulsion organique / eau est réalisée à l'aide des moyens connus de l'homme du métier pour réaliser des émulsions comme par exemple en utilisant un agitateur Ultra Turrax T25 pendant 5 min à 11000tpm.
Cette pré-émulsion est ensuite homogénéisée, à l'aide d'un homogénéisateur haute pression de type OBL20 de chez Niro Soavi, 2 fois à 600 bars pou obtenir des capsules de moins de 1$\mu$m.
Le dichlorométhane est ensuite évaporé à l'aide d'un évaporateur rotatif sous pression réduite.
On obtient ainsi une suspension de nanocapsules contenant 15 % de mélange Y figurant un des composés X ou Y conformément à l'invention. Le contrôle de la taille des nanocapsules est effectué à l'aide d'un granulomètre laser par diffusion de la lumière. La taille moyenne des nanocapsules est de 256 nm.
Ces capsules sont parfaitement stables 2 mois à +4°C, température ambiante et 45 °C.

Exemple 2 :

Préparation de nanocapsules du composé complémentaire de celui de l'exemple 1

| | |
|---|---|
| Phase organique : | |
| Mélange A | 3,62 g |
| Polycaprolactone (Capa®6100, Solvay) | 0,48 g |
| Diméthicone copolyol (DC SH 3773 M, Dow Corning) | 0,24 g |
| Dichlorométhane | 16 mL |
| Phase aqueuse : | |
| Acide N Stéaroyl L glutamique disodique | |
| (Amisoft HS21P d'Ajinomoto) | 0,12 g |
| Eau distillée | 24,14 g |

On suit le même mode opératoire que celui décrit dans l'exemple 1. On obtient une suspension de nanocapsules de 260nm.
Ces capsules sont parfaitement stables 2 mois à +4°C, température ambiante et 45 °C.

Exemple 3 :

Préparation d'une composition selon l'invention

On mélange 50 g de la suspension obtenue à l'exemple 1 avec 50 g de la suspension obtenue à l'exemple 2.
Le mélange reste liquide, on n'observe pas d'évolution dans le temps.
Si on forme un film sur une plaque de verre du mélange précédent, on observe lors de l'évaporation de l'eau, la réticulation des deux silicones réactives. Il y a formation d'un film opalescent, cohésif, pelable.

**Exemple 4 :**

Crème de jour pour le visage, destinée aux peaux sèches

| | |
|---|---|
| Phase A1 : | |
| Distéarate de sucrose commercialisée par la société STERINERIE DUBOIS | 2,00 % |
| Stéarate de sorbitane oxyéthyléné à 4 moles d'oxyde d'éthylène commercialisé sous le nom de TWEEN 61® par la société ICI | 1,40 % |
| Acide stéarique | 0,75 % |
| Vaseline codex | 2,10 % |
| Huile d'avocat | 4,50 % |
| Huile de jojoba | 4,10 % |
| Huile de silicone volatile | 3,70 % |
| Acétate de vitamine E | 0,50 % |
| D $\alpha$ tocophérol commercialisé par la société HENKEL sous le nom de COPHEROL 1300® | 0,30 % |
| Phase A2 : | |
| Parfum | 0,30 % |
| Propylparabène | 0,10 % |
| Phase B : | |
| Glycérine | 5,00 % |
| Méthyl parabène | 0,30 % |
| Triéthanolamine | 0,40 % |
| Eau déminéralisée | q.s.p. 100 % |
| Phase C : | |
| Mélange de polymères carboxyvinyliques commercialisé sous le nom de CARBOPOL 940® par la société NOVEON | 0,30 % |
| Eau déminéralisée | 9,70 % |
| Phase D : | |
| Suspension aqueuse de capsules de l'exemple 1 | 10,00 % |
| Suspension aqueuse de capsules de l'exemple 2 | 10,00 % |

Mode opératoire

La phase huileuse A1 et la phase aqueuse B sont chauffées séparément à la température de 80 °C.

Sous l'agitation de 4000 t/mn fournie par un homogénéisateur Moritz de type Turbo Lab 2100, on verse la phase B sur la phase A et l'on conserve ces conditions d'agitations et de température pendant 30 minutes.

Le mélange est alors introduite dans un homogénéisateur haute pression Soavi de type OBL réglé à la pression de 500 bars pour 3 passages consécutifs. On ajoute alors à l'émulsion la phase huileuse A2 et on soumet le tout à l'agitation donnée par le Turbo Lab 2100 à la vitesse de 3000 t/mn pendant 10 mn. A cette émulsion A1 + B + A2, on ajoute la phase C et l'on agite le tout à l'aide d'un homogénéisateur Rayneri équipé d'une turbine de type défloculeuse à la vitesse de 2500 t/mn pendant 30 mn à la température ambiante. La phase D est ensuite ajoutée sous agitation réduite.

On obtient une émulsion dont l'application sur la peau provoque la rupture des capsules et les composés encapsulés ainsi libérés réagissent pour former un film de polymère sur la peau.

**Exemple 5 :**

Composition solaire

| | |
|---|---|
| Phase A : | |
| Copolymère diglycol cyclohexanediméthanol isophtalates sulfoisophtalates (AQ38S de chez EASTMAN CHEMICAL) | 2,00 % |

(suite)

| | |
|---|---|
| <u>Phase A :</u> | |
| Glycérine | 5,00 % |
| Conservateurs | 1,20 % |
| Séquestrants | 0,10 % |
| Eau déminéralisée | 34,70 % |
| <u>Phase B :</u> | |
| 2-cyano-3,3-diphénylacryltate de 2-éthyl hexyle 4-tertiobutyl 4'-méthoxy dibenzoylméthane (Parsol 1789 vendu par la société GIVAUDAN) | 10,00 %<br>2,00 % |
| Cyclométhicone | 4,00 % |
| Cire liquide de jojoba (Flora Tech) | 4,00 % |
| <u>Phase C :</u> | |
| Gomme de xanthane | 0,50 % |
| Eau déminéralisée q.s.p. | 100,00 % |
| <u>Phase D :</u> | |
| Suspension aqueuse de capsules de l'exemple 1 | 10,00 % |
| Suspension aqueuse de capsule de l'exemple 2 | 10,00 % |

<u>Mode opératoire</u>

On mélange les constituants de la phase A et on chauffe le mélange à 70 °C sous agitation magnétique jusqu'à dispersion complète du polymère, puis on refroidit la solution jusqu'à température ambiante. On prépare par ailleurs la phase B.

On introduit la phase A dans la phase B sous vive agitation. On homogénéise l'émulsion sous une pression de 600 bars (2 à 4 passages) en ramenant l'émulsion à la température ambiante entre chaque passage.

Les phases C et D sont ensuite ajoutées à l'aide d'un homogénéisateur Rayneri équipé d'une turbine de type défloculeuse.

## Revendications

1. Composition cosmétique destinée au soin et/ou au maquillage de matière(s) kératinique(s) comprenant dans un milieu physiologiquement acceptable au moins un composé X, un composé Y et au moins un catalyseur, avec au moins un des composés X ou Y étant un polyorganosiloxane, et lesdits composés X et Y étant susceptibles de réagir ensemble par une réaction d'hydrosilylation en présence d'un catalyseur, avec au moins un composé parmi les composés X et Y étant présent dans ladite composition sous une forme encapsulée, ledit catalyseur étant associé à au moins l'un des composés X ou Y encapsulé.

2. Composition selon la revendication 1 dans laquelle les composés X et Y sont tous deux présents sous des formes encapsulées séparées.

3. Composition selon la revendication 1 ou 2 dans laquelle la forme encapsulée est une nanocapsule de type coeur/écorce dont le coeur de nature lipophile est formé en tout ou partie soit d'au moins un composé X, soit d'au moins un composé Y, soit d'une huile contenant au moins un catalyseur, ledit catalyseur étant encapsulé avec le composé X ou le composé Y.

4. Composition selon la revendication 3 dans laquelle l'écorce des nanocapsules est de nature polymérique.

5. Composition selon l'une des revendications 3 et 4 dans laquelle l'écorce est de nature polymérique, non réticulée, non hydrosoluble et non soluble dans le coeur desdites nanocapsules.

6. Composition selon l'une des revendications 4 et 5 dans laquelle le polymère de l'écorce présente un point de fusion inférieur à 100 °C.

7. Composition selon l'une des revendications 4 à 6 dans laquelle l'écorce des nanocapsules comprend au moins un

polymère choisi parmi :

- les polymères de cyanoacrylate d'alkyle en $C_2$-$C_{12}$,
- les poly-L-lactides, les poly-DL-lactides, les polyglycolides et les copolymères correspondants,
- les polycaprolactones,
- les polymères de l'acide 3-hydroxy butyrique,
- les copolymères de chlorure de vinyle et d'acétate de vinyle,
- l'acéto-phtalate de polyvinyle,
- l'acéto-phtalate de cellulose,
- le copolymère polyvinylpyrrolidone-acétate de vinyle,
- les polyéthylènevinylacétates,
- les copolymères d'acide et d'ester méthacrylique,
- les polyacrylonitriles,
- les polyacrylamides,
- les polyéthylèneglycols,
- les poly-(méthacrylate d'hydroxyalkyle en $C_1$ à $C_4$),
- les dérivés de cellulose,
- le polystyrène et ses copolymères,
- les oligomères styrène alkylalcool,
- les terpolymères d'éthylène, d'acétate de vinyle et d'anhydride maléique,
- les polyamides,
- les polyéthylènes,
- les polypropylènes,
- les organopolysiloxanes,
- les poly (alkylène adipate),
- les polyesters polyol,
- les polymères siliconés de polysilsesquioxane,
- les polyesters dendritiques à fonction hydroxyle terminale, et
- leurs mélanges.

**8.** Composition selon l'une des revendications 4 à 7, dans laquelle le polymère de l'écorce des nanocapsules est choisi parmi :

- les polycaprolactones,
- l'acéto-phtalate de polyvinyle,
- l'acéto-phtalate de cellulose,
- les copolymères d'acide et d'ester méthacrylique,
- les dérivés de cellulose,
- le polystyrène et ses copolymères,
- les polyamides,
- les organopolysiloxanes,
- les poly (alkylène adipate), et
- leurs mélanges.

**9.** Composition selon l'une des revendications 4 à 8, dans laquelle l'écorce des nanocapsules comprend au moins un polymère choisi parmi les polycaprolactones.

**10.** Composition selon l'une des revendications 3 à 9 dans laquelle les nanocapsules ont un diamètre inférieur à 1 μm, de préférence inférieur ou égal à 500 nm, par exemple compris entre 200 et 500 nm, de préférence inférieur ou égal à 350 nm, par exemple compris entre 200 et 350 nm.

**11.** Composition selon l'une quelconque des revendications précédentes, dans laquelle le composé X est choisi parmi les composés siliconés comprenant au moins deux groupements aliphatiques insaturés.

**12.** Composition selon la revendication 11 dans laquelle le composé X est un polyorganosiloxane comprenant une chaîne principale siliconée dont les groupements aliphatiques insaturés sont pendants à la chaîne principale (groupe latéral) ou situés aux extrémités de la chaîne principale du composé (groupe terminal).

**13.** Composition selon la revendication 12, dans laquelle le composé X est porteur d'au moins un groupe polaire.

**14.** Composition selon l'une quelconque des revendications précédentes, dans laquelle le composé X est choisi parmi les polyorganosiloxanes comprenant au moins deux groupements aliphatiques insaturés liés chacun à un atome de silicium

**15.** Composition selon l'une quelconques des revendications précédentes, dans laquelle le composé X est choisi parmi les polyorganosiloxanes comprenant des unités siloxanes de formule :

$$R_m R'SiO_{\frac{(3-m)}{2}} \quad (I)$$

dans laquelle :

- R représente un groupe hydrocarboné monovalent, linéaire ou cyclique, comprenant de 1 à 30 atomes de carbone,
- m est égal à 1 ou 2 et
- R' représente :

  un groupement hydrocarboné aliphatique insaturé comprenant de 2 à 10, de préférence de 3 à 5 atomes de carbone ou un groupement hydrocarboné cyclique insaturé comprenant de 5 à 8 atomes de carbone.

**16.** Composition selon la revendication 15 dans laquelle le polyorganosiloxane de formule (I) est tel que R' représente un groupe vinyle ou un groupe -R"-CH=CHR"' dans laquelle R" est une chaine hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène.

**17.** Composition selon la revendication 15 ou 16, dans laquelle R représente un radical alkyle comprenant de 1 à 10 atomes de carbone ou encore un groupement phényle, et de préférence un radical méthyle, et R' est un groupe vinyle.

**18.** Composition selon l'une quelconque des revendications précédentes, dans laquelle les polyorganosiloxanes comprennent en outre des unités de formule

$$R_n SiO_{\frac{(4-n)}{2}} \quad (II)$$

dans laquelle R représente un groupe hydrocarboné monovalent, linéaire ou cyclique, comprenant de 1 à 30 atomes de carbone, et n est égal à 1, 2 ou 3.

**19.** Composition selon l'une quelconque des revendications 1 à 10, dans laquelle le composé X est choisi parmi les oligomères ou polymères organiques, les oligomères ou polymères hybrides organique/silicone, lesdits oligomères ou polymères portant au moins 2 groupements aliphatiques insaturés réactifs.

**20.** Composition selon l'une des revendications précédentes, dans laquelle le composé Y comprend au moins deux groupes Si-H libres.

**21.** Composition selon l'une des revendications précédentes, dans laquelle le composé Y est choisi parmi les polyorganosiloxanes comprenant au moins une unité alkylhydrogènosiloxanes de formule suivante :

$$R_pHSiO_{\frac{(3-p)}{2}} \quad (III)$$

dans laquelle :

R représente un groupe hydrocarboné monovalent, linéaire ou cyclique, comprenant de 1 à 30 atomes de carbone ou un groupe phényle, et p est égal à 1 ou 2.

**22.** Composition selon la revendication précédente, dans laquelle le composé Y est tel que les radicaux R représentent un groupement alkyle en $C_1$-$C_{10}$, de préférence méthyle.

**23.** Composition selon l'une des revendications 20 à 22, dans laquelle Y est un polyorganosiloxane comprenant au moins deux unités alkylhydrogènosiloxane de formule -$(H_3C)(H)Si$-O- et comprenant éventuellement des unités -$(H_3C)_2SiO$-

**24.** Composition selon l'une des revendications précédentes, dans laquelle l'hydrosilylation est réalisée en présence d'un catalyseur à base de platine ou d'étain.

**25.** Composition selon la revendication précédente, dans laquelle le catalyseur est présent en une teneur allant de 0,0001 % à 20 % en poids par rapport au poids total de la composition le comprenant.

**26.** Composition selon l'une quelconque des revendications 1 à 20 , dans laquelle le composé X est un polydiméthylsiloxane à groupements vinyliques terminaux et le composé Y est un polyméthylhydrogénosiloxane.

**27.** Composition selon l'une quelconque des revendications précédentes, dans laquelle le catalyseur est encapsulé avec un composé X choisi parmi les polyorganosiloxanes comprenant au moins deux groupements aliphatiques insaturés.

**28.** Composition selon l'une quelconque des revendications précédentes dans laquelle le composé X est porteur d'au moins un groupe polaire susceptible de former une liaison hydrogène avec les matières kératiniques.

**29.** Composition selon l'une quelconque des revendications précédentes comprenant en outre au moins une charge choisie parmi la silice ou la silice traitée en surface.

**30.** Composition selon l'une des revendications précédentes, dans laquelle le composé X présente une masse moléculaire moyenne en poids (Mw) allant de 150 à 1 000 000, de préférence de 200 à 800 000, de préférence encore de 200 à 250 000.

**31.** Composition selon l'une des revendications précédentes, dans laquelle le composé Y présente une masse moléculaire en poids (Mw) allant de 200 à 1 000 000, de préférence de 300 à 800 000, de préférence encore de 500 à 250 000.

**32.** Composition selon l'une des revendications précédentes, dans laquelle le composé X représente de 0,5 % à 95 % en poids par rapport au poids total de la composition, de préférence de 1 % à 90 % et mieux de 5 % à 80 %.

**33.** Composition selon l'une des revendications précédentes, dans laquelle le composé Y représente de 0,5 % à 95 % en poids par rapport au poids total de la composition, de préférence de 1 % à 90 % et mieux de 5 % à 80 %.

**34.** Composition selon l'une des revendications précédentes, dans laquelle les composés X et Y sont présents dans la composition en un ratio molaire X/Y allant de 0,05 à 20 et mieux de 0,1 à 10.

**35.** Composition selon l'une des revendications précédentes, comprenant une phase grasse liquide.

**36.** Composition selon la revendication précédente, dans laquelle la phase grasse liquide comprend au moins une huile.

**37.** Composition selon la revendication précédente, dans laquelle la ou les huiles sont présentes en une teneur allant de 1 % à 90 % en poids, de préférence de 5 % à 50 % en poids par rapport au poids total de la composition.

**38.** Composition selon l'une quelconque des revendications précédentes, comprenant en outre au moins un composé choisi parmi les vitamines, les épaississants, les gélifiants, les oligo-éléments, les adoucissants, les séquestrants, les parfums, les agents alcalinisants ou acidifiants, les conservateurs, les parfums, les filtres solaires, les tensioactifs, les anti-oxydants, les fibres, les charges, les matières colorantes, les polymères filmogènes, les cires, les actifs cosmétiques comme les actifs bactéricides ou anti-transpirants, les neutralisants, les émollients, les hydratants et leurs mélanges.

**39.** Composition selon l'une des revendications précédentes, **caractérisée en ce qu'**il s'agit d'une composition de revêtement de la peau du corps ou du visage, plus particulièrement d'une composition de maquillage ou de soin de la peau du corps ou du visage.

**40.** Procédé cosmétique de revêtement pour le soin et/ou le maquillage de matière(s) kératinique(s) comprenant au moins l'application sur ladite matière kératinique d'un mélange de composés X et Y susceptibles de réagir ensemble par une réaction d'hydrosilylation en présence d'un catalyseur, ledit mélange étant issu d'une composition cosmétique comprenant, dans un milieu physiologiquement acceptable, au moins un tel composé X, un tel composé Y et au moins un catalyseur, et dans laquelle au moins un composé parmi les composés X, Y et le catalyseur, est présent dans ladite composition sous une forme encapsulée.

**41.** Procédé selon la revendication précédente, dans lequel le mélange est obtenu soit de manière extemporanée avant application sur ladite matière kératinique, soit au moment de l'application de ladite composition sur ladite matière kératinique.

**42.** Procédé selon la revendication 40 ou 41, dans lequel la composition est telle que définie dans l'une quelconque des revendications 1 à 39.

**43.** Utilisation d'une forme encapsulée d'au moins un composé X, d'une forme encapsulée d'au moins un composé Y et/ou d'une forme encapsulée d'au moins un catalyseur pour la préparation d'une composition de soin et/ou de maquillage de matière(s) kératinique(s) comprenant un milieu physiologiquement acceptable et destinée à former, après application et séchage sur ladite matière kératinique, un film polymérique issu de la réaction d'un composé X et d'un composé Y selon une réaction de type hydrosilylation en présence d'un catalyseur, avec au moins l'un des composés X ou Y étant un polyorganosiloxane.

**44.** Utilisation selon la revendication précédente, dans laquelle la composition est telle que définie dans l'une quelconque des revendications 1 à 39.

**45.** Composition cosmétique destinée au soin et/ou au maquillage de matière(s) kératinique(s) comprenant dans un milieu physiologiquement acceptable au moins un composé X et un composé Y, et éventuellement un catalyseur, avec au moins un des composés X et Y étant un polyorganosiloxane, et lesdits composés X et Y étant susceptibles de réagir ensemble par une réaction de condensation, avec au moins un composé parmi les composés X, Y et le catalyseur lorsqu'il est présent, étant présent dans ladite composition sous une forme encapsulée.

Office européen des brevets

# RAPPORT DE RECHERCHE EUROPEENNE

Numéro de la demande
EP 07 12 3549

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,Y | GB 2 051 102 A (TORAY SILICONE CO) 14 janvier 1981 (1981-01-14) * revendications; exemples * ----- | 43,45 | INV. A61Q5/06 A61Q5/12 A61K8/895 A61K8/89 A61K8/11 |
| X,Y | EP 0 300 645 A2 (DOW CORNING [US]) 25 janvier 1989 (1989-01-25) * revendications; exemples * ----- | 1-45 | |
| X,Y | EP 0 550 239 A1 (DOW CORNING [US]) 7 juillet 1993 (1993-07-07) * revendications 1,2; exemples * ----- | 1-45 | |
| X,Y | DATABASE WPI Week 199210 Derwent Publications Ltd., London, GB; AN 1992-077274 XP002444112 & JP 04 023867 A (KANEGAFUCHI CHEM KK) 28 janvier 1992 (1992-01-28) * abrégé * ----- | 43,45 | |
| X,Y | DATABASE WPI Week 200239 Derwent Publications Ltd., London, GB; AN 2002-355777 XP002444113 & JP 2002 012768 A (DOW CORNING TORAY SILICONE CO LTD) 15 janvier 2002 (2002-01-15) * abrégé * ----- | 43-45 | DOMAINES TECHNIQUES RECHERCHES (IPC) A61K A61Q |
| Y | EP 1 552 820 A (OREAL [FR]) 13 juillet 2005 (2005-07-13) * revendications; exemples * ----- | 1-45 | |
| Y | WO 01/54658 A (PROCTER & GAMBLE [US]) 2 août 2001 (2001-08-02) * page 5, ligne 14 - ligne 17; revendications * ----- -/-- | 1-45 | |

Le présent rapport a été établi pour toutes les revendications

| Lieu de la recherche | Date d'achèvement de la recherche | Examinateur |
|---|---|---|
| Munich | 4 mars 2008 | KRATTINGER, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

Office européen des brevets

# RAPPORT DE RECHERCHE EUROPEENNE

Numéro de la demande
EP 07 12 3549

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | WO 2006/028612 A (DOW CORNING [US]; MURPHY KEVIN PATRICK [US]; SCHALAU GERALD KENNETH II) 16 mars 2006 (2006-03-16)<br>* alinéas [0008], [0012], [0013], [0074], [0075] *<br>----- | 1-45 | |
| P,X | WO 2007/071885 A (OREAL [FR]; JAEGER LEZER NATHALIE [FR]) 28 juin 2007 (2007-06-28)<br>* page 4, ligne 26 - page 5, ligne 7 *<br>----- | 1-45 | |
| P,X | WO 2007/071886 A (OREAL [FR]; BENABDILLAH KATARINA [FR]; COTHIAS PASCALE [FR]) 28 juin 2007 (2007-06-28)<br>* page 33 - ligne 13; revendications *<br>----- | 1-45 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| Munich | 4 mars 2008 | KRATTINGER, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 07 12 3549

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

04-03-2008

| Document brevet cité au rapport de recherche | | Date de publication | Membre(s) de la famille de brevet(s) | | Date de publication |
|---|---|---|---|---|---|
| GB 2051102 | A | 14-01-1981 | AU | 531902 B2 | 08-09-1983 |
| | | | AU | 5898080 A | 11-12-1980 |
| | | | BE | 883617 A1 | 03-12-1980 |
| | | | BR | 8003510 A | 05-01-1981 |
| | | | CA | 1135438 A1 | 09-11-1982 |
| | | | DE | 3020858 A1 | 18-12-1980 |
| | | | FR | 2458574 A1 | 02-01-1981 |
| | | | IT | 1148869 B | 03-12-1986 |
| | | | JP | 1411282 C | 24-11-1987 |
| | | | JP | 55161849 A | 16-12-1980 |
| | | | JP | 62016977 B | 15-04-1987 |
| | | | SE | 8004133 A | 05-12-1980 |
| | | | US | 4293677 A | 06-10-1981 |
| EP 0300645 | A2 | 25-01-1989 | AU | 602103 B2 | 27-09-1990 |
| | | | AU | 1914588 A | 27-01-1989 |
| | | | CA | 1338199 C | 02-04-1996 |
| | | | DE | 3852229 D1 | 12-01-1995 |
| | | | DE | 3852229 T2 | 29-06-1995 |
| | | | JP | 1051140 A | 27-02-1989 |
| | | | JP | 1866439 C | 26-08-1994 |
| | | | JP | 5070495 B | 05-10-1993 |
| | | | US | 4874667 A | 17-10-1989 |
| EP 0550239 | A1 | 07-07-1993 | CA | 2085804 A1 | 03-07-1993 |
| | | | DE | 69222137 D1 | 16-10-1997 |
| | | | DE | 69222137 T2 | 02-04-1998 |
| | | | JP | 3110906 B2 | 20-11-2000 |
| | | | JP | 5271546 A | 19-10-1993 |
| | | | US | 5279898 A | 18-01-1994 |
| | | | US | 5194460 A | 16-03-1993 |
| JP 4023867 | A | 28-01-1992 | AUCUN | | |
| JP 2002012768 | A | 15-01-2002 | AUCUN | | |
| EP 1552820 | A | 13-07-2005 | FR | 2864900 A1 | 15-07-2005 |
| | | | JP | 2005248162 A | 15-09-2005 |
| WO 0154658 | A | 02-08-2001 | AU | 3649701 A | 07-08-2001 |
| | | | US | 6274127 B1 | 14-08-2001 |
| WO 2006028612 | A | 16-03-2006 | CN | 101010065 A | 01-08-2007 |
| | | | EP | 1793811 A1 | 13-06-2007 |
| | | | KR | 20070049653 A | 11-05-2007 |

EPO FORM P0460

Pour tout renseignement concernant cette annexe : voir Journal Officiel de l'Office européen des brevets, No.12/82

## ANNEXE AU RAPPORT DE RECHERCHE EUROPEENNE RELATIF A LA DEMANDE DE BREVET EUROPEEN NO.

EP 07 12 3549

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

04-03-2008

| Document brevet cité au rapport de recherche | Date de publication | Membre(s) de la famille de brevet(s) | Date de publication |
|---|---|---|---|
| WO 2007071885 A | 28-06-2007 | WO 2007071706 A2 | 28-06-2007 |
| WO 2007071886 A | 28-06-2007 | FR 2894817 A1 | 22-06-2007 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0196450 A
- GB 2407496 A
- FR 1583243
- WO 0209862 A
- WO 9308908 A
- WO 02051536 A
- EP 274961 A
- EP 1552820 A
- FR 2836381
- FR 2790405
- FR 2787729
- EP 447318 A
- US 5364633 A
- US 5411744 A
- FR 2742677 A
- EP 0465744 A
- EP 959066 A
- EP 1057849 A
- WO 9317060 A
- WO 9612754 A
- US 3175993 A
- US 4772675 A
- US 4871827 A
- US 4888380 A
- US 4898910 A
- US 4906719 A
- US 4962174 A
- EP 705593 A
- US 4874554 A
- US 4137180 A
- EP 1172089 A
- US 4234435 A
- US 4708753 A
- US 5129972 A
- US 4931110 A
- GB 2156799 A
- US 4877756 A
- US 4919179 A
- EP 864320 A
- EP 1661550 A


### Littérature non-brevet citée dans la description


- **EKWALL.** Adv. Liq. Cryst. 1968
- **ROSERVEAR.** *JSCC,* 1968, vol. 19, 581
- **LACHAMPT ; VILA.** *Revue Française des Corps Gras,* 1969, 87-111
- **DONALD A. TOMALIA et al.** *Angewandte Chemie, Int. Engl. Ed.,* vol. 29 (2), 138-175
- **KUSABE.M.** *Pitture e Verniei - European Coating,* 2005, vol. 12-B, 43-49
- **PROBSTER, M.** *Adhesion-Kleben & Dichten,* 2004, vol. 481 (1-2), 12-14
- **KUSABE.M.** *Pitture e Verniei - European Coating,* 2005, vol. 12-B, 43-49
- **PROBSTER, M.** *Adhesion-Kleben & Dichten,* 2004, vol. 481 (1-2), 12-14
- **LANDON, S.** *Pitture e Verniei,* 1997, vol. 73 (11), 18-24
- **HUANG, MOWO.** *Pitture e Verniei,* 2000, vol. 5, 61-67
- **KIRK-OTHMER.** Encyclopedia of Chemical Technology. WILEY, 1979, vol. 22, 333-432
- **ALLGAIER ; POPPE ; WILLNER ; RICHTER.** *Macromolecules,* 1997, vol. 30, 1582-1586